# EUROPEAN PATENT APPLICATION

(11) **EP 4 400 095 A1**
(43) Date of publication of application: **17.07.2024**
(21) Application number: 24151951.1
(22) Date of filing: 15.01.2024
(51) Int. Cl.: A61K 9/00, A61K 31/353, A61K 47/10, A61K 47/12, A61K 47/26, A61K 47/36, A61K 47/40, A61P 31/04, A61P 31/12, A61P 31/14

(54) **EGCG BUCCAL TABLET AND USE THEREOF**

(30) Priority: 16.01.2023 CN 202310064585; 06.12.2023 CN 202311661466
(71) Applicant: Hainan University, Hainan 570228 (CN)
(72) Inventor: YANG, Jingjing, HAIKOU, 570228 (CN)
(74) Representative: Botti & Ferrari S.p.A.

(57) **Abstract**

The present application relates to the use of EGCG in the manufacture of a product for preventing and/or treating viral infections or bacterial infections, and also relates to a buccal tablet containing EGCG, and also relates to the use of the buccal tablet in the manufacture of a product for preventing and/or treating viral infections or bacterial infections.

## Description

The present application is based on and claims the benefits of priorities from Chinese application No. 202310064585.2 filed on January 16, 2023 and Chinese application No. 202311661466.1 filed on December 06, 2023, the disclosures of which are incorporated herein by reference in their entirety.

### Technical Field

The present application relates to EGCG buccal tablets and uses thereof.

### Background Art

EGCG (Epigallocatechin gallate) has a structural formula as follows:

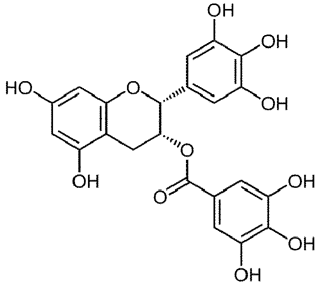

EGCG is one of the components of green tea polyphenols. It is a catechin monomer isolated from tea leaves, and has antibacterial, antiviral, antioxidation, anti-arteriosclerosis, anti-thrombosis, anti-angiogenesis, antiinflammatory and anti-tumor functions. In the antiviral researches of EGCG (Int. J. Mol. Sci. 2022, 23, 9842; Journal of Virology, 2014, 88(14):7806-7817; Phytomedicine, 2021(85): 153286; CN 105535012 B), it is found that EGCG shows antiviral activity against SARS-Cov-2, vesicular stomatitis virus (VSV), human immunodeficiency virus (HIV), influenza virus (such as IAV), enterovirus 71, adenovirus (AdV), hepatitis B virus (HBV), herpes simplex virus types 1 and 2 (HSV-1 and HSV-2), hepatitis C virus (HCV), and other viruses. In the mechanism researches (Int. J. Mol. Sci. 2022, 23, 9842; Journal of Virology, 2014, 88(14):7806-7817), it is further found that EGCG mainly competes with heparin sulfate and sialic acid that are highly expressed in the cells of respiratory tract and digestive tract for binding virus particles, thereby inhibiting the attachment of these viruses to the cells. For example, some viruses such as HSV and SARS-CoV-2 use heparin sulfate as a receptor to infect cells, while other viruses such as influenza A virus (IAV) and adenovirus (AdV) use cells expressing sialic acid as receptor to infect the body. This provides the possibility for EGCG to be developed to antiviral drugs that target virus attachment during the virus invasion to the host.

On the other hand, the infectious diseases caused by respiratory viruses or other viruses transmitted through the respiratory tract are common clinical diseases. In particular, the sudden outbreak of COVID-19 caused by the SARS-CoV-2 virus poses a huge threat to global public health. There are many types of viruses, such as coronavirus, influenza virus, adenovirus, rhinovirus, parainfluenza virus, respiratory syncytial virus, enterovirus, that cause respiratory and pulmonary infections. These viruses mainly enter the human body through the mouth and nose when breathing, to invade the nose, pharynx and larynx, and cause inflammation and even systemic symptoms. The buccal cavity and nasal cavity are the main ways for respiratory viruses to invade the body. Taking COVID-19 as an example, currently existing vaccines cannot prevent the transmission of SARS-CoV-2 virus, and there is an urgent need to develop new means of post-exposure prevention and treatment.

EGCG has been developed to fight infections. Some literature (Acta Laser Biology, 2020, 29(5): 392-397) discloses that EGCG is used to treat infections caused by influenza viruses, in which it is mainly administered orally and enters the blood circulation to exert its medicinal effect. However, EGCG is a highly polar substance with eight phenolic hydroxyl groups in its structure, so it cannot be well absorbed by the body when routinely administered systemically, and even if it is absorbed into the body, it will take a long time to take effect. Because it is extremely unstable and easily degraded, the administration of EGCG is difficult to achieve systemic antiviral effects. In a study (Drug Metab Dispos, 1997, 25, 1045-1050), it is found that the bioavailability of EGCG was only 0.1% when rats were intragastrically administered with decaffeinated green tea (DGT) (200 mg/kg) enriched with EGCG.

In summary, there are currently no literature reports on the use of EGCG monomer and formulation thereof for the prevention and treatment of viral infections in the buccal cavity and throat.

### Contents of the Invention

The inventors unexpectedly found that EGCG could quickly inactivate viruses locally, it did not need to be absorbed, but only being sucked in the buccal cavity, and it performed local and effective inactivation of respiratory viruses in the buccal cavity and throat where respiratory viruses entry and invade the body. The inventors further unexpectedly found that EGCG at a concentration of 0.2mM to 15.06mM could quickly and effectively inactivate influenza A virus, influenza B virus, novel enterovirus (such as EV-D68), common coronavirus, SARS-CoV-2 and other viruses, which transmitted through the respiratory tract, within a short period of time (1 to 20 minutes, preferably 1 to 5 minutes, more preferably 1 minute). It was unexpectedly found in further simulation experiments at the animal level that even if the viruses that were rapidly inactivated by EGCG in the buccal cavity continued to invade the human body, the viral loads in the nose and lung which were important infected tissues were significantly reduced, which further confirmed that EGCG could locally inactivate viruses in the buccal cavity and bring about an unexpected surprise effect.

The inventors also unexpectedly found that normally, the basic volume of saliva an adult secreted was 0.5ml per minute, and it could be seen that 0.046 to 3.5mg of EGCG (with molecular weight of 458.37) was administrated buccally in the buccal cavity for 1 minute, its locally average concentration in buccal cavity could reach 0.2 to 15.28mM for inactivating viruses. On this basis, the inventors further prepared EGCG buccal tablets with a dosage range of 0.3 mg to 100 mg (preferably 5 to 10 mg). The buccal tablets had sufficient disintegration and dissolution time (1 to 13.5 minutes) and could be dissolved to provide enough amount of EGCG. The average dissolving time of the buccal tablet in the buccal cavity of the subjects was 4 minutes and 19 seconds, and the average chewing and dissolving time of the buccal tablet in the buccal cavity was 17.7 seconds, which ensured that the buccal tablet could be diluted and dissolved in the buccal cavity with saliva in 1 to 5 minutes to release EGCG with sufficient effective concentration in the buccal cavity to inactivate viruses in the buccal cavity and throat, thereby preventing the transmission and infection of viruses.

EGCG has eight phenolic hydroxyl groups that can be easily oxidized and are extremely unstable. The inventors further unexpectedly found that adding fructo-oligosaccharides to EGCG buccal tablets could significantly improve the stability of the buccal tablets, significantly increase the compressibility of the buccal tablets, and make the buccal tablets to have excellent taste.

The inventors further unexpectedly found that EGCG could locally and rapidly inactivate *Helicobacter pylori* with a minimum bactericidal concentration (MBC) of 200 µM.

Based on the above findings, the present application was completed.

The present application provides a use of EGCG in the manufacture of a product for preventing and/or treating a viral infection or a bacterial infection, wherein EGCG is administered buccally, and the residence time of EGCG in the buccal cavity is greater than or equal to 1 minute.

The present application also provides a composition for preventing and/or treating a viral infection or a bacterial infection, which comprises EGCG, wherein EGCG is administered buccally, and the residence time of EGCG in the buccal cavity is greater than or equal to 1 minute.

The present application also provides EGCG for use in preventing and/or treating a viral infection or a bacterial infection, wherein EGCG is administered buccally, and the residence time of EGCG in the buccal cavity is greater than or equal to 1 minute.

The present application also provides a method for preventing and/or treating a viral infection or a bacterial infection, which comprises administering buccally an effective amount of EGCG to a subject in need thereof, and the residence time of EGCG in the buccal cavity is greater than or equal to 1 minute.

The present application also provides a use of EGCG in the manufacture of a product for preventing and/or treating a bacterial infection, wherein EGCG is administered buccally, and the residence time of EGCG in the buccal cavity is greater than or equal to 1 minute.

The present application also provides a composition for preventing and/or treating a bacterial infection, which comprises EGCG, wherein EGCG is administered buccally, and the residence time of EGCG in the buccal cavity is greater than or equal to 1 minute.

The present application also provides EGCG for use in preventing and/or treating a bacterial infection, wherein EGCG is administered buccally, and the residence time of EGCG in the buccal cavity is greater than or equal to 1 minute.

The present application also provides a method for preventing and/or treating a bacterial infection, which comprises administering buccally an effective amount of EGCG to a subject in need thereof, and the residence time of EGCG in the buccal cavity is greater than or equal to 1 minute.

The present application also provides a use of EGCG in the manufacture of a product for preventing and/or treating a viral infection, wherein EGCG is administered buccally, and the residence time of EGCG in the buccal cavity is greater than or equal to 1 minute.

The present application also provides a composition for preventing and/or treating a viral infection, which comprises EGCG, wherein EGCG is administered buccally, and the residence time of EGCG in the buccal cavity is greater than or equal to 1 minute.

The present application also provides EGCG for use in preventing and/or treating a viral infection, wherein EGCG is administered buccally, and the residence time of EGCG in the buccal cavity is greater than or equal to 1 minute.

The present application also provides a method for preventing and/or treating a viral infection, which comprises administering buccally an effective amount of EGCG to a subject in need thereof, and the residence time of EGCG in the buccal cavity is greater than or equal to 1 minute.

In some embodiments, the residence time of EGCG in the buccal cavity is 1 to 20 minutes, preferably 1 to 5 minutes. In some embodiments, the average concentration of EGCG locally in the buccal cavity is 0.2 to 16mM. In some embodiments, the average concentration of EGCG locally in the buccal cavity is 0.2 to 15.5mM. In some embodiments, the average concentration of EGCG in the buccal cavity is 0.2 to 15.28mM. In some embodiments, the average concentration of EGCG locally in the buccal cavity is 0.2 to 15mM. In some embodiments, the average concentration of EGCG locally in the buccal cavity is 0.2 to 12mM. In some embodiments, the average concentration of EGCG locally in the buccal cavity is 0.2 to 10 mM. In some embodiments, the average concentration of EGCG locally in the buccal cavity is 0.2 to 8mM. In some embodiments, the average concentration of EGCG locally in the buccal cavity is 0.2 to 6mM. In some embodiments, the average concentration of EGCG locally in the buccal cavity is 0.2 to 5mM. In some embodiments, the average concentration of EGCG locally in the buccal cavity is 0.2 to 4mM. In some embodiments, the average concentration of EGCG locally in the buccal cavity is 0.2 to 2mM.

In some embodiments, the virus infection is an infection caused by a respiratory virus or other virus that is transmitted through the respiratory tract. In some embodiments, the virus infection is an infection caused by a virus selected from the group consisting of influenza A virus, influenza B virus, rhinovirus (HRV), adenovirus (AdV), coronavirus (including but not limited to common coronavirus, SARS virus, SARS-CoV-2), vesicular stomatitis virus (VSV), human immunodeficiency virus (HIV), enterovirus 71 (EV71), hepatitis B virus (HBV), herpes simplex virus type 1 and 2 (HSV-1 and HSV-2), hepatitis C virus (HCV), coxsackie virus, and novel enterovirus D68 (EV-D68). In some embodiments, the virus infection is an infection caused by a SARS-CoV-2.

In some embodiments, the bacterium infection is an infection caused by a *Helicobacter pylori.*

In some embodiments, EGCG exists in the form of a buccal tablet.

In some embodiments, the product comprises food, medicament, health product, and the like.

In some embodiments, the buccal tablet comprises EGCG, a filler, a lubricant, and a flavoring agent, and the EGCG content in each buccal tablet is 0.3 mg to 100 mg.

In some embodiments, the buccal tablet further comprises a coloring agent. In some embodiments, the coloring agent is a food coloring.

In some embodiments, the buccal tablet further comprises an adhesive.

In some embodiments, the filler is one or more selected from the group consisting of cyclodextrin, lactose, mannitol, sorbitol, glycine, microcrystalline cellulose, starch, skimmed milk powder, collagen, and dextrin.

In some embodiments, the cyclodextrin is one or more selected from the group consisting of α-cyclodextrin, β-cyclodextrin, γ-cyclodextrin and pharmaceutically acceptable cyclodextrin derivative. In some embodiments, the pharmaceutically acceptable cyclodextrin derivative is one or more selected from the group consisting of dimethyl-β-cyclodextrin, 2-hydroxyethyl-β-cyclodextrin, 2-hydroxypropyl-β-cyclodextrin, 3-hydroxypropyl-β-cyclodextrin, sulfobutylether-β-cyclodextrin and trimethyl-β-cyclodextrin. In some embodiments, the cyclodextrin is one or more selected from the group consisting of β-cyclodextrin, dimethyl-β-cyclodextrin, 2-hydroxyethyl-β-cyclodextrin, 2-hydroxypropyl-β-cyclodextrin, 3-hydroxypropyl-β-cyclodextrin, sulfobutylether-β-cyclodextrin and trimethyl-β-cyclodextrin. According to the "Chinese Pharmacopoeia", unless otherwise specified, hydroxypropyl-β-cyclodextrin in the present application refers to 2-hydroxypropyl-β-cyclodextrin.

In some embodiments, the lubricant is one or more selected from the group consisting of magnesium stearate, talc, and silicon dioxide.

In some embodiments, the flavoring agent is one or more selected from the group consisting of sweetening agent, menthol, fruity flavoring agent, and souring agent. In some embodiments, the flavoring agent includes sweetening agent, and menthol. In some embodiments, the flavoring agent includes sweetening agent, menthol, and fruity flavoring agent. In some embodiments, the flavoring agent includes sweetening agent, menthol, and souring agent. In some embodiments, the flavoring agent includes sweetening agent, menthol, fruity flavoring agent, and souring agent. In some embodiments, the sweetening agent is one or more selected from the group consisting of sucrose, sucralose, glucose, aspartame, xylitol, sorbitol, fructose, fructo-oligosaccharides, and stevioside. In some embodiments, the sweetening agent is fructo-oligosaccharides. In some embodiments, the sweetening agent is a combination of fructo-oligosaccharides and other sweetening agent, and the other sweetening agent is one or more selected from the group consisting of sucrose, sucralose, glucose, aspartame, xylitol, fructose and stevioside. In some embodiments, the souring agent is one or more selected from the group consisting of L-malic acid, D-malic acid, and DL-malic acid. In some embodiments, the fruity flavoring agent includes, but is not limited to, fruity flavouring, fruit powder, and the like. In some embodiments, the fruity flavouring includes, but is not limited to, strawberry flavouring, orange flavouring, lemon flavouring, cherry flavouring, apple flavouring, etc. In some embodiments, the fruit powder includes, but is not limited to, apple, pineapple, mango, banana, strawberry, papaya, orange, and pear fruit powders.

In some embodiments, the adhesive is one or more selected from the group consisting of sodium carboxymethylcellulose, methylcellulose, povidone, tragacanth, and gum arabic.

In some embodiments, the EGCG content in each buccal tablet is 0.5 mg to 50 mg, such as 0.625 mg, 1.25 mg, 2.5 mg, 5 mg, 7.5 mg, 10 mg, 25 mg, or 50 mg.

In some embodiments, the EGCG content in each buccal tablet is 2.5 mg to 7.5 mg. In some embodiments, the EGCG content in each buccal tablet is 5 mg to 10 mg.

In some embodiments, the residence time of the buccal tablet in the buccal cavity is greater than or equal to 1 minute. In some embodiments, the residence time of the buccal tablet in the buccal cavity is 1 to 20 minutes. In some embodiments, the residence time of the buccal tablet in the buccal cavity is 1 to 5 minutes, or 5 to 15 minutes, or 15 to 20 minutes.

In some embodiments, the buccal tablet is dissolved by greater than 70% within 15 to 20 minutes when measured by the paddle method at 37°C and 100 rpm in an aqueous medium.

Normal saliva is colorless, odorless, nearly neutral (pH value is 6.6 to 7.1), and its main component is water, accounting for 98.5% to 99%. Degassed purified water at 37 °C ± 1 °C can be used to simulate oral saliva; the disintegration time of the buccal tablet is determined though the determination method of disintegration time of the Chinese Pharmacopoeia, 2020 Edition, and the dissolution time of the buccal tablet in the subject's buccal cavity is recorded at the same time. The experimental results of multiple batches show that: there is an obvious correspondence between the disintegration time and the dissolution time of the buccal tablet provided by the present application in the human buccal cavity. When the disintegration time is less than 10 minutes, the buccal tablet will be completely dissolved in the human buccal cavity within 10 minutes. For example, the average dissolution time of a batch of buccal tablets in the buccal cavities of 6 subjects was 4 minutes and 19 seconds, and the disintegration time of this batch of buccal tablets in the disintegration instrument was 8 minutes and 44 seconds. For another example, the average dissolution time of another batch of buccal tablets in the buccal cavities of the subjects was 5 minutes and 02 seconds, and the disintegration time of this batch of buccal tablets in the disintegration instrument was 10 minutes and 54 seconds.

In some embodiments, the filler content in each buccal tablet is 20 to 200 times the EGCG content. In some embodiments, the filler content in each buccal tablet is 30 to 150 times the EGCG content. In some embodiments, the filler content in each buccal tablet is 40 to 100 times the EGCG content. In some embodiments, the filler content in each buccal tablet is 40 to 160 times the EGCG content. In some embodiments, the filler content in each buccal tablet is 60 to 120 times the EGCG content. In some embodiments, the filler content in each buccal tablet is 80 to 140 times the EGCG content.

In some embodiments, the lubricant content in each buccal tablet is 0.1 to 5.0% by weight of the buccal tablet. In some embodiments, the lubricant content in each buccal tablet is 0.5 to 3.5% by weight of the buccal tablet. In some embodiments, the lubricant content in each buccal tablet is 1 to 3% by weight of the buccal tablet. In some embodiments, the lubricant content in each buccal tablet is 1.5 to 2.5% by weight of the buccal tablet.

In some embodiments, the flavoring agent content in each buccal tablet is 0.5 to 100 times the EGCG content. In some embodiments, the flavoring agent content in each buccal tablet is 1 to 100 times the EGCG content. In some embodiments, the flavoring agent content in each buccal tablet is 1 to 50 times the EGCG content. In some embodiments, the flavoring agent content in each buccal tablet is 1 to 15 times the EGCG content. In some embodiments, the flavoring agent content in each buccal tablet is 5 to 25 times the EGCG content. In some embodiments, the flavoring agent content in each buccal tablet is 10 to 35 times the EGCG content. In some embodiments, the flavoring agent content in each buccal tablet is 5 to 20 times the EGCG content.

In some embodiments, the flavoring agent comprises a sweetening agent and menthol, wherein the menthol content in each buccal tablet is 0.01 to 5 times the EGCG content. In some embodiments, the menthol content in each buccal tablet is 0.1 to 4 of the EGCG content. In some embodiments, the menthol content in each buccal tablet is 0.2 to 3 times the EGCG content. In some embodiments, the menthol content in each buccal tablet is 0.01 to 1.5 times the EGCG content. In some embodiments, the menthol content in each buccal tablet is 0.05 to 1 times the EGCG content. In some embodiments, the menthol content in each buccal tablet is 0.1 to 0.75 times the EGCG content.

In some embodiments, the flavoring agent further comprises a fruity flavoring agent, wherein the content of the fruity flavoring agent in each buccal tablet is no more than 30 times the EGCG content. In some embodiments, the content of the fruity flavoring agent in each buccal tablet is no more than 25 times the EGCG content. In some embodiments, the content of the fruity flavoring agent in each buccal tablet is no more than 20 times the EGCG content. In some embodiments, the content of the fruity flavoring agent in each buccal tablet is 15 times the EGCG content. In some embodiments, the content of the fruity flavoring agent in each buccal tablet is 10 times the EGCG content.

In some embodiments, the flavoring agent further comprises a souring agent, wherein the content of the souring agent in each buccal tablet is 0.01 to 7 times the EGCG content. In some embodiments, the content of the souring agent in each buccal tablet is 0.01 to 1.5 times the EGCG content. In some embodiments, the content of the souring agent in each buccal tablet is 0.05 to 1 times the EGCG content. In some embodiments, the content of the souring agent in each buccal tablet is 0.1 to 1 times the EGCG content. In some embodiments, the content of the souring agent in each buccal tablet is 0.05 to 4 times the EGCG content. In some embodiments, the content of the souring agent in each buccal tablet is 0.1 to 2 times the EGCG content.

In some embodiments, the sweetening agent comprises fructo-oligosaccharides, wherein the content of fructo-oligosaccharides in each buccal tablet is 2 to 25 times the EGCG content. In some embodiments, the content of fructo-oligosaccharides in each buccal tablet is 3 to 20 times the EGCG content. In some embodiments, the content of fructo-oligosaccharides in each buccal tablet is 4 to 15 times the EGCG content.

In some embodiments, the content of the adhesive in each buccal tablet is 0.1 to 5.0% by weight of the buccal tablet. In some embodiments, the content of the adhesive in each buccal tablet is 0.5% to 3.5% by weight of the buccal tablet. In some embodiments, the content of the adhesive in each buccal tablet is 0.5% to 2.5% by weight of the buccal tablet.

In some embodiments, the buccal tablet contains:

| | |
|---|---|
| EGCG | 2.0% by weight |
| Filler | 91.0% by weight |
| Lubricant | 2.0% by weight |
| Flavoring agent | 3.0% by weight |
| Adhesive | 2.0% by weight. |

In some embodiments, the buccal tablet contains:

| | |
|---|---|
| EGCG | 2.0% by weight |
| Filler | 89.5% by weight |
| Lubricant | 2.0% by weight |
| Flavoring agent | 4.5% by weight |
| Adhesive | 2.0% by weight. |

In some embodiments, the buccal tablet contains:

| | |
|---|---|
| EGCG | 1.0% by weight |
| Filler | 50.4% by weight |
| Lubricant | 2.0% by weight |
| Flavoring agent | 45.6% by weight |
| Adhesive | 1.0% by weight. |

In some embodiments, the buccal tablet contains:

| | |
|---|---|
| EGCG | 1.0% by weight |
| Filler | 88.4% by weight |
| Lubricant | 2.0% by weight |
| Flavoring agent | 7.6% by weight |
| Adhesive | 1.0% by weight. |

In some embodiments, the buccal tablet contains:

| | |
|---|---|
| EGCG | 1.0% by weight |
| Filler | 87.9% by weight |
| Lubricant | 2.0% by weight |
| Flavoring agent | 7.6% by weight |
| Adhesive | 1.0% by weight |
| Food coloring | 0.5% by weight. |

In some embodiments, the buccal tablet contains:

| | |
|---|---|
| EGCG | 1.0% by weight |
| Filler | 87.0% by weight |
| Lubricant | 2.0% by weight |
| Flavoring agent | 9.5% by weight |
| Food coloring | 0.5% by weight. |

In some embodiments, the buccal tablet contains:

| | |
|---|---|
| EGCG | 1.0% by weight |
| Filler | 84.2% by weight |
| Lubricant | 2.0% by weight |
| Flavoring agent | 12.3% by weight |
| Food coloring | 0.5% by weight. |

In some embodiments, the buccal tablet contains:

| | |
|---|---|
| EGCG | 0. 7∼1.5 % by weight |
| Sorbitol | 77∼82% by weight |
| fructo-oligosaccharides | 3∼7% by weight |
| Sucralose | 0.3∼1.5% by weight |
| Menthol | 0.2∼0.4% by weight |
| DL-malic acid | 0.3∼1% by weight |
| Aspartame | 1∼2% by weight |
| Magnesium stearate | 1∼3% by weight |
| Fruit flavoring | 8∼10% by weight |
| Food coloring | 0.02∼0.2% by weight. |

In some embodiments, the buccal tablet contains:

| | |
|---|---|
| Sorbitol | 60.48% by weight |
| Fruity flavoring agent | 15.00% by weight |
| Skimmed milk powder | 15.00% by weight |
| fructo-oligosaccharides | 5.00% by weight |
| EGCG | 0.83% by weight |
| Magnesium stearate | 2.00% by weight |
| Aspartame | 0.20% by weight |
| Sucralose | 0.50% by weight |
| DL-malic acid | 0.80% by weight |
| Menthol | 0.10% by weight |
| Food coloring | 0.09% by weight. |

In some embodiments, the buccal tablet contains:

| | |
|---|---|
| Sorbitol | 49.38% by weight |
| Fruity flavoring agent | 20.00% by weight |
| Skimmed milk powder | 20.00% by weight |
| fructo-oligosaccharides | 5.00% by weight |
| EGCG | 0.83% by weight |
| Magnesium stearate | 3.00% by weight |
| Aspartame | 0.20% by weight |
| Sucralose | 0.60% by weight |
| DL-malic acid | 0.80% by weight |
| Menthol | 0.10% by weight |
| Food coloring | 0.09% by weight. |

In some embodiments, the buccal tablet contains:

| | |
|---|---|
| Sorbitol | 74.30% by weight |
| Fruity flavoring agent | 9.00% by weight |
| Skimmed milk powder | 8.00% by weight |
| fructo-oligosaccharides | 5.00% by weight |
| Magnesium stearate | 2.00% by weight |
| EGCG | 0.71% by weight |
| Sucralose | 0.25% by weight |
| DL-malic acid | 0.50% by weight |
| Aspartame | 0.10% by weight |
| Menthol | 0.05% by weight |
| Food coloring | 0.09% by weight. |

In some embodiments, the buccal tablet of the present application has a hardness greater than or equal to 70N. In some embodiments, the buccal tablet of the present application has a hardness of 70N to 230N.

The present application also provides a buccal tablet, which is the buccal tablet described in any of the preceding embodiments of the present application.

The present application also provides a buccal tablet, which comprises EGCG, a filler, a lubricant, and a flavoring agent, and each buccal tablet contains 0.3 mg to 100 mg EGCG.

The present application also provides a use of the buccal tablet in the manufacture of a product for preventing and/or treating a viral infection or a bacterial infection.

The present application also provides a use of the buccal tablet in the manufacture of a product for preventing and/or treating a bacterial infection.

The present application also provides a use of the buccal tablet in the manufacture of a product for preventing and/or treating a viral infection.

In some embodiments, the virus infection is an infection caused by a respiratory virus or other virus that is transmitted through the respiratory tract. In some embodiments, the virus infection is an infection caused by a virus selected from the group consisting of influenza A virus, influenza B virus, rhinovirus (HRV), adenovirus

(AdV), coronavirus (including but not limited to common coronavirus, SARS virus, SARS-CoV-2), vesicular stomatitis virus (VSV), human immunodeficiency virus (HIV), enterovirus 71 (EV71), hepatitis B virus (HBV), herpes simplex virus 1 and 2 (HSV-1 and HSV-2), hepatitis C virus (HCV), coxsackie virus, and novel enterovirus D68 (EV-D68). In some embodiments, the virus infection is an infection caused by a SARS-CoV-2.

In some embodiments, the bacterium infection is an infection caused by a *Helicobacter pylori.*

### Definition of Terms

In the present application, unless otherwise specified, the scientific and technical terms used herein have the meanings generally understood by those skilled in the art. Meanwhile, in order to better understand the present application, the definitions and explanations of relevant terms are provided as follows.

As used herein, the term "buccal tablet" generally refers to a compressed tablet that dissolves in the buccal cavity, can produce medicinal effect on the buccal cavity and pharynx, and can be sucked directly or sucked after chewing.

As used herein, the term "food coloring" refers to a type of coloring agent, which is a type of substance that can be eaten in moderate amounts by humans and can change the original color of product (e.g., food, medicament, and health product) to a certain extent. The food coloring can be of natural origin or synthetic.

As used herein, the term "fruity flavouring" refers to a flavouring with natural fruity flavour that is formulated with natural or natural equivalent flavorings and synthetic flavorings with reference to the natural fruity falvour.

As used herein, the term "fruit powder" refers to a substance made from fruits as raw materials by processing (for example, by freeze-drying and other processes). The fruit powder can be a single raw material fruit powder or a composite raw material fruit powder.

As used herein, the term "fructo-oligosaccharide (FOS)" refers to a functional oligosaccharide such as kestose, nystose and kestopentaose fructofuranosylnystose that are produced by bounding 1 to 3 fructose groups to the fructose group in sucrose through β (2 → 1) glycosidic linkage, which are a natural active ingredient and an excellent water-soluble dietary fiber (Chinese Food Additives, 22,1:11-15). As the most potential functional oligosaccharide and sweetening agent, fructo-oligosaccharides have been used in dairy products, lactobacillus beverages, solid beverages, candies, biscuits, bread, jelly, cold drinks and other foods. As an excellent prebiotic, the polysaccharide component of fructo-oligosaccharides has been proven to have effects of relieving mood, regulating intestinal flora, enhancing immunity, promoting mineral absorption, improving lipid metabolism, and lowering blood sugar (Chinese Food Additives, 22,1:11-15; Acta Pharmaceutica Sinica B, 2022, 12(8):3298-3312; China Journal of Chinese Materia Medica, 1995, 20(1): 36-39). At the same time, fructo-oligosaccharides are a pure natural sweetening agent with good taste, the sweetness thereof is 0.3 to 0.6 times that of sucrose. They not only maintain the pure sweetness of sucrose, but are also sweeter and refreshing than sucrose, and thus can be used to replace part of sucrose in the production of various candies, jelly, chocolate and other products, which can not only maintain a certain sweetness, but also prevent and treat dental caries, and are especially suitable for children (Chinese Food Additives, 22, 1: 11-15).

As used herein, the term "effective amount" refers to an amount sufficient to achieve a desired preventive or therapeutic effect, for example, an amount that achieves alleviation of a symptom associated with a disease to be treated, or an amount to effectively avoid, reduce, prevent or delay the occurrence of disease. The determination of such an effective amount is within the capabilities of those skilled in the art.

As used herein, the terms "treating" and "treatment" are used interchangeably and intended to alleviate, relieve, ameliorate, or eliminate a targeted disease state or condition. If a subject receives a therapeutic amount of the buccal tablets according to the method described herein, one or more indications and symptoms of the subject show observable and/or detectable reduction or improvement, the subject is successfully "treated". It should also be understood that the treatment of a disease state or condition comprises not only the disease state or condition is complete treated, but also although the disease state or condition is not completed treated, some biological or medical related results are achieved.

As used herein, the terms "preventing" and "prevention" are used interchangeably and intended to avoid, reduce, prevent or delay the occurrence of a disease or a disease-associated symptom before such disease or disease-associated symptom occurs prior to the administration of relevant drug. "Prevention" does not necessarily require completely preventing the occurrence of the disease or disease-associated symptom. For example, if the administration of relevant drug can reduce the risk of a subject to develop a specific disease or disease-associated symptom, or alleviate the severity of subsequent associated symptoms, it can be considered as "preventing" the occurrence or development of the disease.

Unless otherwise apparent from the context, all values provided herein are modified by the term "about," and the term "about" shall be understood to be within the normal tolerance range in the art, for example, "about" shall be understood to be within ±10%, ±9%, ±8%, ±7%, ±6%, ±5%, ±4%, ±3%, ±2%, ±1%, ±0.5%, ±0.1%, ±0.05% or ±0.01% of the value.

### Beneficial Effects

The present application has one or more of the following advantages:
1) EGCG can quickly inactivate viruses locally, it do not need to be absorbed, but only being sucked in the buccal cavity, and it performs local and effective inactivation of respiratory viruses in the buccal cavity and throat where respiratory viruses entry and invade the body. EGCG at a concentration of 0.2mM to 15.06mM can quickly and effectively inactivate respiratory viruses within a short period of time (1 to 20 minutes, preferably 1 to 5 minutes, more preferably 1 minute). It is found in further simulation experiments at the animal level that even if the viruses that have been rapidly inactivated by EGCG in the buccal cavity continue to invade the human body, the viral loads in the nose and lung which were important infected tissues are significantly reduced.
2) The buccal tablets provided by the present application exert their medicinal effects rapidly, can disintegrate and dissolve in the buccal cavity in a short period of time, and can effectively and quickly inactivate viruses in the buccal cavity and throat. EGCG (with molecular weight of 458.37) at a concentration of 0.2mM to 15.06mM can effectively and quickly inactivate SARS-CoV-2, influenza A virus, influenza B virus and other viruses within 1 to 20 minutes and not need to be absorbed. Normally, the basic volume of saliva an adult secreted is 0.5ml per minute, 0.046 to 1.145mg of EGCG administrated buccally in the buccal cavity for 1 minute can reach an locally average concentration of 0.2 to 5 mM; 0.046 to 3.5mg of EGCG administrated buccally in the buccal cavity for 1 minute can reach an locally average concentration of 0.2 to 15.28 mM; and the EGCG at all of above concentrations of can effectively inactivate the viruses. The buccal tablets of the present application containing 0.3 mg to 100 mg of EGCG (preferably 5 to 10 mg) have sufficient disintegration time and dissolution time, and thus can ensure that the EGCG released after disintegration of the buccal tablets has sufficient effective concentration and residence time in buccal cavity after being diluted with saliva to inactivate the viruses in the buccal cavity and throat, thereby preventing the transmission and infection of the viruses.
3) The buccal tablets provided by the present application are easy to carry and use, can be eaten repeatedly at any time, and have broad application prospects in the prevention and treatment after virus exposure. The buccal tablets of the present application are particularly suitable for post-exposure prophylaxis for people who are in close contact with influenza, COVID-19, etc., and people who live in the same household with virus infection cases, so they have a wide range of application scenarios. When there is a risk during travel or gathering indoors, the buccal tablets can be taken by one piece at any time or can be taken repeatedly.
4) The buccal tablets provided by the present application have good taste and fruity flavor, and are especially suitable for high-risk groups such as children and teenagers. Children have special characteristics in the development of their immune systems and are relatively vulnerable to viral infections, especially in autumn and winter, and are highly susceptible to superinfection with multiple viruses such as SARS-CoV-2, influenza virus, and respiratory syncytial virus (RSV). According to data from Centers for Disease Control and Prevention, as of August 2022, The probability of infants under the age of 6 months in the U.S. hospitalized with the coronavirus are about the same as those in the United States who are 65 to 74 years old. The total number of children in China is huge, with approximately 253.38 million children aged 0-14 years old, accounting for 17.95% of the total population. The buccal tablets of the present application, especially the fruit-flavored buccal tablets, are particularly suitable for consumption by children and adolescents, and can exert the broad-spectrum inactivation effect of EGCG on respiratory viruses.
5) The buccal tablets provided by the present application are safe enough and can be taken repeatedly per day. EGCG is an active ingredient in tea, and it has been approved as a new resource food in the Announcement No. 17 of the Ministry of Health in October 2010 and is allowed to be used in ordinary food. According to the strict toxicological assessment on new resource food safety from the Ministry of Health, the maximum intake of EGCG can reach 300 mg/day. The buccal tablet of the present application contains 0.3 mg to 100 mg of active ingredient EGCG, which are safe enough and can be consumed repeatedly within one day.
6) The buccal tablets provided by the present application can be used for prevention and treatment before and after viral infection.

### Brief Description of the Drawings

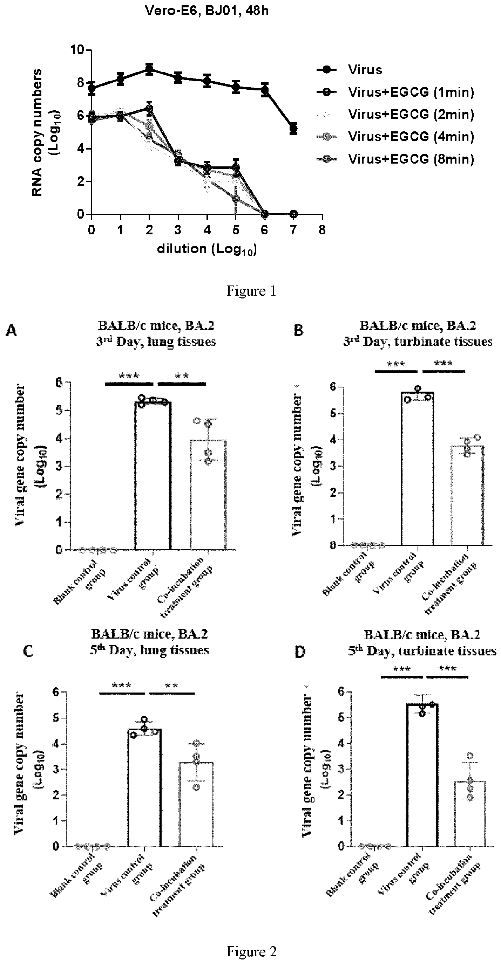

Figure 1 shows the determination results of virus copy number in the cell supernatant after Vero-E6 cells were infected by the co-incubation solution produced by co-incubating EGCG with SARS-CoV-2 BJ-01 virus for 48 hours. The results showed that after SARS-CoV-2 BJ-01 was co-incubated with 2.18 mM EGCG for 1 minute, 2 minutes, 4 minutes and 8 minutes at room temperature, the number of live viruses was significantly reduced, suggesting that 2.18 mM EGCG could quickly and effectively inactivate SARS-CoV-2 within just 1 minute.
Figure 2 shows the determination results of viral titers and viral loads in the tissues of turbinates and lungs of mice on 3^{rd} day or 5^{th} day after the mice were challenged with the co-incubation solution produced by co-incubating EGCG with SARS-CoV-2 Omicron BA.2 virus. The results showed that the nucleic acid loads of SARS-CoV-2 Omicron BA.2 in the lungs and turbinates of the mice in the EGCG and virus co-incubation group were significantly reduced.

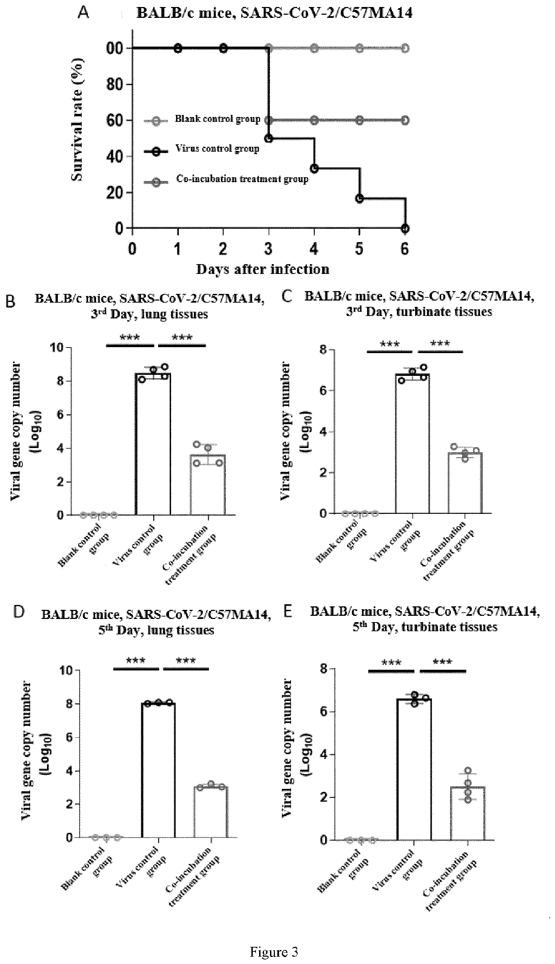

Figure 3 shows the survival status of mice monitored as well as the determination results of viral titers and viral loads in the tissues of turbinates and lungs of mice on the 3^{rd} day or 5^{th} day after the mice were challenged with the co-incubation solution produced by co-incubating EGCG with SARS-CoV-2/C57MA14 virus. The results showed that as compared to the virus control group, the co-incubation of EGCG and virus for 10 minutes could not only protect 60% of mice from death caused by lethal dose SARS-CoV-2 C57MA14 infection (A), but also significantly reduce the viral nucleic acid loads in the tissues of lungs and turbinates of mice on the 3^{rd} day (B and C) and 5^{th} day (D and E) after the challenge.

### Specific Models for Carrying Out the Invention

The embodiments of the present application will be described in detail below with reference to examples, but those skilled in the art will understand that the following examples and experimental examples are only used to illustrate the present application and should not be regarded as limiting the scope of the present application. If the specific conditions are not indicated in the Examples and Experimental examples, they shall be carried out according to the conventional conditions or the conditions recommended by the manufacturer. The reagents or instruments used, the manufacturers of which are not indicated, are all conventional products that can be commercially available.

The EGCG used in the examples of the present application was purchased fromHuzhou Rongkai Foliage Extract Co.,Ltd, sorbitol and sucralose were purchased from Jiangxi Alpha Hi-Tech Pharmaceutical Co., Ltd., strawberry flavouring was purchased from Shangqiu Qingshan Flavors and Fragrances Co., Ltd., fructo-oligosaccharides were purchased from Quantum Hi-Tech (Guangdong) Biological Co., Ltd., magnesium stearate was purchased fromAnhui Sunhere Pharmaceutical Excipients Co.,Ltd., aspartame was purchased from Changzhou Saikesi Chemical Co., Ltd., DL-malic acid was purchased from Changzhou Saikesi Chemical Co., Ltd., menthol was purchased from Zhengzhou Yuhe Food Additive Co., Ltd., allura red lake and lemon yellow lake were purchased from Shanghai Yipin Pigment Co., Ltd., lemon fruit powder and strawberry fruit powder were purchased from Shanghai Fukuan Trading Co., Ltd.

The disintegration time described in the examples of the present application was determined according to the determination method of disintegration time of the Chinese Pharmacopoeia, 2020 Edition, by using degassed purified water at 37 °C ± 1 °C to simulate oral saliva.

### Example 1

| Ingredient | Function | Proportion, % | Prescription dose/mg |
|---|---|---|---|
| EGCG | | 2.00% | 5 |
| Hydroxypropyl-β-cyclodextrin | Filler | 15.00% | 37.5 |
| Mannitol | Filler | 50.00% | 125.0 |
| Lactose | Filler | 26.00% | 65.0 |
| Povidone K30 | Adhesive | 2.00% | 5 |
| Stevioside | Flavoring agent | 1.50% | 3.75 |
| Menthol | Flavoring agent | 1.50% | 3.75 |
| Magnesium stearate | Lubricant | 2.00% | 5 |
| Tablet weight | / | 100% | 250 |

According to the prescription in the table, EGCG was mixed with other ingredients, passed through a 40-mesh sieve, and pressed into tablets (round tablets, with hardness of 70N). After tasting, the tablets had good taste and a very faint bitterness could be felt. The disintegration time of the tablets was measured by a disintegration instrument and was 5 minutes and 28 seconds.

### Example 2

| Ingredient | Function | Proportion, % | Prescription dose/mg |
|---|---|---|---|
| EGCG | | 2.00% | 5 |
| Hydroxypropyl-β-cyclodextrin | Filler | 13.5% | 33.75 |
| Mannitol | Filler | 50.00% | 125.0 |
| Lactose | Filler | 26.00% | 65.0 |
| Povidone K30 | Adhesive | 2.00% | 5 |
| Stevioside | Flavoring agent | 3.00% | 7.50 |
| Menthol | Flavoring agent | 1.50% | 3.75 |
| Magnesium stearate | Lubricant | 2.00% | 5 |
| Tablet weight | / | 100% | 250 |

According to the prescription in the table, EGCG was mixed with other ingredients, passed through a 40-mesh sieve, and pressed into tablets (round tablets, with hardness of 70N). After tasting, the tablets had good taste and a very faint bitterness (which was slightly aggravated as compared to Example 1) could be felt. The disintegration time of the tablets was measured by a disintegration instrument and was 5 minutes and 15 seconds.

### Example 3

| Ingredient | Function | Proportion, % | Prescription dose/mg |
|---|---|---|---|
| EGCG | | 1.00% | 5 |
| Hydroxypropyl-β-cyclodextrin | Filler | 15.00% | 75 |
| Mannitol | Filler | 24.00% | 120 |
| Lactose | Filler | 11.4% | 57 |
| Povidone K30 | Adhesive | 1.00% | 5 |
| Sucrose | Flavoring agent | 40.00% | 200 |
| Stevioside | Flavoring agent | 0.50% | 2.5 |
| Menthol | Flavoring agent | 0.10% | 0.5 |
| Magnesium stearate | Lubricant | 2.00% | 10 |
| Tangerine flavouring | Flavoring agent | 5.00% | 25 |
| Tablet weight | / | 100% | 500 |

According to the prescription in the table, EGCG was mixed with other ingredients, passed through a 40-mesh sieve, and pressed into tablets (special-shaped tablets, with hardness of 90N). After tasting, the tablets had no bitterness, and the overall taste was good. The disintegration time of the tablets was measured by a disintegration instrument and was about 7 to 8 minutes.

### Example 4

| Ingredient | Function | Proportion, % | Prescription dose/mg |
|---|---|---|---|
| EGCG | | 1.00% | 5 |
| Hydroxypropyl-β-cyclodextrin | Filler | 15.00% | 75 |
| Mannitol | Filler | 24.00% | 120 |
| Lactose | Filler | 11.4% | 57 |
| Povidone K30 | Adhesive | 1.00% | 5 |
| Sucrose | Flavoring agent | 40.00% | 200 |
| Stevioside | Flavoring agent | 0.50% | 2.5 |
| Menthol | Flavoring agent | 0.10% | 0.5 |
| Magnesium stearate | Lubricant | 2.00% | 10 |
| Lemon flavouring | Flavoring agent | 5.00% | 25 |
| Tablet weight | / | 100% | 500 |

According to the prescription in the table, EGCG was mixed with other ingredients, passed through a 40-mesh sieve, and pressed into tablets (special-shaped tablets, with hardness of 90N). After tasting, the tablets had no bitterness, and the overall taste was good. The disintegration time of the tablets was measured by a disintegration instrument and was about 7 to 8 minutes.

### Example 5

| Ingredient | Function | Proportion, % | Prescription dose/mg |
|---|---|---|---|
| EGCG | | 1.00% | 5 |
| Hydroxypropyl-β-cyclodextrin | Filler | 15.00% | 75 |
| Mannitol | Filler | 24.00% | 120 |
| Lactose | Filler | 11.4% | 57 |
| Povidone K30 | Adhesive | 1.00% | 5 |
| Sucrose | Flavoring agent | 40.00% | 200 |
| Stevioside | Flavoring agent | 0.50% | 2.5 |
| Menthol | Flavoring agent | 0.10% | 0.5 |
| Magnesium stearate | Lubricant | 2.00% | 10 |
| Strawberry flavouring | Flavoring agent | 5.00% | 25 |
| Tablet weight | / | 100% | 500 |

According to the prescription in the table, EGCG was mixed with other ingredients, passed through a 40-mesh sieve, and pressed into tablets (special-shaped tablets, with hardness of 90N). After tasting, the tablets had no bitterness, and the overall taste was good. The disintegration time of the tablets was measured by a disintegration instrument and was about 7 to 8 minutes.

### Example 6

| Ingredient | Function | Proportion, % | Prescription dose/mg |
|---|---|---|---|
| EGCG | | 1.00% | 5 |
| Hydroxypropyl-β-cyclodextrin | Filler | 15.00% | 75 |
| Mannitol | Filler | 62.50% | 312.5 |
| Lactose | Filler | 10.90% | 54.5 |
| Povidone K30 | Adhesive | 1.00% | 5 |
| Stevioside | Flavoring agent | 1.50% | 7.5 |
| Menthol | Flavoring agent | 0.10% | 0.5 |
| Magnesium stearate | Lubricant | 2.00% | 10 |
| Strawberry flavouring | Flavoring agent | 6.00% | 30 |
| Tablet weight | / | 100% | 500 |

According to the prescription in the table, EGCG was mixed with other ingredients, passed through a 40-mesh sieve, and pressed into tablets (special-shaped tablets, with hardness of 70N). After tasting, the tablets had no bitterness, and the overall taste was good. The disintegration time of the tablets was measured by a disintegration instrument and was about 8 minutes.

### Example 7

| Ingredient | Function | Proportion, % | Prescription dose/mg |
|---|---|---|---|
| EGCG | | 1.00% | 5 |
| Hydroxypropyl-β-cyclodextrin | Filler | 15.00% | 75 |
| Mannitol | Filler | 62.50% | 312.5 |
| Lactose | Filler | 10.90% | 54.5 |
| Povidone K30 | Adhesive | 1.00% | 5 |
| Stevioside | Flavoring agent | 1.50% | 7.5 |
| Menthol | Flavoring agent | 0.10% | 0.5 |
| Magnesium stearate | Lubricant | 2.00% | 10 |
| Orange flavouring | Flavoring agent | 6.00% | 30 |
| Tablet weight | / | 100% | 500 |

According to the prescription in the table, EGCG was mixed with other ingredients, passed through a 40-mesh sieve, and pressed into tablets (special-shaped tablets, with hardness of 70N). After tasting, the tablets had no bitterness, and the overall taste was good. The disintegration time of the tablets was measured by a disintegration instrument and was about 8 minutes.

### Example 8

| Ingredient | Function | Proportion, % | Prescription dose/mg |
|---|---|---|---|
| EGCG | | 1.00% | 5 |
| Hydroxypropyl-β-cyclodextrin | Filler | 15.00% | 75 |
| Mannitol | Filler | 62.50% | 312.5 |
| Lactose | Filler | 10.90% | 54.5 |
| Povidone K30 | Adhesive | 1.00% | 5 |
| Stevioside | Flavoring agent | 1.50% | 7.5 |
| Menthol | Flavoring agent | 0.10% | 0.5 |
| Magnesium stearate | Lubricant | 2.00% | 10 |
| Cherry flavouring | Flavoring agent | 6.00% | 30 |
| Tablet weight | / | 100% | 500 |

According to the prescription in the table, EGCG was mixed with other ingredients, passed through a 40-mesh sieve, and pressed into tablets (special-shaped tablets, with hardness of 70N). After tasting, the tablets had no bitterness, and the overall taste was good. The disintegration time of the tablets was measured by a disintegration instrument and was about 8 minutes.

### Example 9

| Ingredient | Function | Proportion, % | Prescription dose/mg |
|---|---|---|---|
| EGCG | | 1.00% | 5 |
| Hydroxypropyl-β-cyclodextrin | Filler | 15.00% | 75 |
| Mannitol | Filler | 62.00% | 310 |
| Lactose | Filler | 10.90% | 54.5 |
| Povidone K30 | Adhesive | 1.00% | 5 |
| Stevioside | Flavoring agent | 1.50% | 7.5 |
| Menthol | Flavoring agent | 0.10% | 0.5 |
| Magnesium stearate | Lubricant | 2.00% | 10 |
| Strawberry flavouring | Flavoring agent | 6.00% | 30 |
| Orange coloring agent | Coloring agent | 0.50% | 2.5 |
| Tablet weight | / | 100.0% | 500.0 |

According to the prescription in the table, EGCG was mixed with other ingredients, passed through a 40-mesh sieve, and pressed into tablets (special-shaped tablets, with hardness of 70N). After tasting, the tablets had no bitterness, and the overall taste was good. The disintegration time of the tablets was measured by a disintegration instrument and was about 8 minutes. The product had an orange appearance and good visual effects.

### Example 10

| Ingredient | Function | Proportion, % | Prescription dose/mg |
|---|---|---|---|
| EGCG | | 1.00% | 5 |
| Mannitol | Filler | 70.00% | 350 |
| Lactose | Filler | 17.00% | 85 |
| Sucralose | Flavoring agent | 1.50% | 7.5 |
| Stevioside | Flavoring agent | 1.50% | 7.5 |
| Menthol | Flavoring agent | 0.50% | 2.5 |
| Magnesium stearate | Lubricant | 2.00% | 10 |
| Lemon flavouring | Flavoring agent | 6.00% | 30 |
| Orange coloring agent | Coloring agent | 0.50% | 2.5 |
| Tablet weight | / | 100.00% | 500 |

According to the prescription in the table, EGCG was mixed with other ingredients, passed through a 40-mesh sieve, and pressed into tablets (special-shaped tablets, with hardness of 100N). After tasting, the tablets had no bitterness, but a refreshing taste, and the overall taste was good. The disintegration time of the tablets was measured by a disintegration instrument and was about 10 minutes. The product had an orange appearance and good visual effects.

### Example 11

| Ingredient | Function | Proportion, % | Prescription dose/mg |
|---|---|---|---|
| EGCG | | 1.00% | 5 |
| Mannitol | Filler | 70.00% | 350 |
| Lactose | Filler | 17.00% | 85 |
| Sucralose | Flavoring agent | 2.50% | 12.5 |
| Stevioside | Flavoring agent | 0.50% | 2.5 |
| Menthol | Flavoring agent | 0.50% | 2.5 |
| Magnesium stearate | Lubricant | 2.00% | 10 |
| Lemon flavouring | Flavoring agent | 6.00% | 30 |
| Orange coloring agent | Coloring agent | 0.50% | 2.5 |
| Tablet weight | / | 100.00% | 500 |

According to the prescription in the table, EGCG was mixed with other ingredients, passed through a 40-mesh sieve, and pressed into tablets (special-shaped tablets, with hardness of 100N). After tasting, the tablets had no bitterness, but a refreshing taste, and the overall taste was good. The disintegration time of the tablets was measured by a disintegration instrument and was about 10 minutes. The product had an orange appearance and good visual effects.

### Example 12

| Ingredient | Function | Proportion, % | Prescription dose/mg |
|---|---|---|---|
| EGCG | | 1.00% | 5 |
| Mannitol | Filler | 70.00% | 350 |
| Lactose | Filler | 17.00% | 85 |
| Sucralose | Flavoring agent | 0.50% | 2.5 |
| Stevioside | Flavoring agent | 2.50% | 12.5 |
| Menthol | Flavoring agent | 0.50% | 2.5 |
| Magnesium stearate | Lubricant | 2.00% | 10 |
| Lemon flavouring | Flavoring agent | 6.00% | 30 |
| Orange coloring agent | Coloring agent | 0.50% | 2.5 |
| Tablet weight | / | 100.00% | 500 |

According to the prescription in the table, EGCG was mixed with other ingredients, passed through a 40-mesh sieve, and pressed into tablets (with a hardness of 150N or 225N). After tasting, the tablets had a refreshing taste, the overall taste was good, but they were slightly bitter as compared to the product of Example 11. The product had an orange appearance and good visual effects.

The disintegration time of the product with a hardness of 150N was about 13 minutes; and the disintegration time of the product with a hardness of 225N was about 13.5 minutes.

### Example 13

| Ingredient | Function | Proportion, % | Prescription dose/mg |
|---|---|---|---|
| EGCG | | 1.00% | 5 |
| Sorbitol | Filler | 84.20% | 421 |
| Fructo-oligosaccharides | Flavoring agent | 5.00% | 25 |
| Sucralose | Flavoring agent | 1.50% | 7.5 |
| Menthol | Flavoring agent | 0.30% | 1.5 |
| L-malic acid | Flavoring agent | 0.50% | 2.5 |
| Aspartame | Flavoring agent | 2.00% | 10 |
| Magnesium stearate | Lubricant | 2.00% | 10 |
| Strawberry flavouring | Flavoring agent | 3.00% | 15 |
| Orange coloring agent | Coloring agent | 0.50% | 2.5 |
| Tablet weight | / | 100.00% | 500 |

According to the prescription in the table, EGCG was mixed with other ingredients, passed through a 40-mesh sieve, and pressed into tablets (with hardness of 210N). After tasting, the tablets had good taste, and a refreshing feel. The disintegration time of the tablets was measured by a disintegration instrument and was about 11.5 minutes. The product had an orange appearance and good visual effects.

### Example 14

| | Prescription 1 | | Prescription 2 | | Prescription 3 | | Prescription 4 | |
|---|---|---|---|---|---|---|---|---|
| Ingredient | Proportion % | Prescription dose/mg | Proportion % | Prescription dose/mg | Proportion % | Prescription dose/mg | Proportion % | Prescription dose/mg |
| EGCG | 0.71% | 5.00 | 0.71% | 5.00 | 0.71% | 5.00 | 0.71% | 5.00 |
| Sorbitol | 86.89% | 608.20 | 85.89% | 601.20 | 83.89% | 587.20 | 81.89% | 573.20 |
| Fructooligosacchar ides | 0.00% | 0.00 | 1.00% | 7.00 | 3.00% | 21.00 | 5.00% | 35.00 |
| Sucralose | 0.50% | 3.50 | 0.50% | 3.50 | 0.50% | 3.50 | 0.50% | 3.50 |
| Menthol | 0.30% | 2.10 | 0.30% | 2.10 | 0.30% | 2.10 | 0.30% | 2.10 |
| DL-malic acid | 0.50% | 3.50 | 0.50% | 3.50 | 0.50% | 3.50 | 0.50% | 3.50 |
| Aspartame | 1.00% | 7.00 | 1.00% | 7.00 | 1.00% | 7.00 | 1.00% | 7.00 |
| Magnesium stearate | 2.00% | 14.00 | 2.00% | 14.00 | 2.00% | 14.00 | 2.00% | 14.00 |
| Strawberry flavouring | 8.00% | 56.00 | 8.00% | 56.00 | 8.00% | 56.00 | 8.00% | 56.00 |
| Allura red lake | 0.09% | 0.63 | 0.09% | 0.63 | 0.09% | 0.63 | 0.09% | 0.63 |
| Lemon yellow lake | 0.01% | 0.07 | 0.01% | 0.07 | 0.01% | 0.07 | 0.01% | 0.07 |
| Tablet weight | 100% | 700.00 | 100% | 700.00 | 100% | 700.00 | 100% | 700.00 |

According to the prescriptions in the table, EGCG was mixed with other ingredients, passed through a 40-mesh sieve, and pressed into tablets.

### Example 15

| Ingredient | Proportion, % | Prescription dose/mg |
|---|---|---|
| EGCG | 0.71% | 5 |
| Sorbitol | 81.93% | 573.48 |
| Fructo-oligosaccharides | 5.00% | 35.00 |
| Sucralose | 0.50% | 3.50 |
| Menthol | 0.30% | 2.10 |
| DL-malic acid | 0.50% | 3.50 |
| Aspartame | 1.00% | 7.00 |
| Magnesium stearate | 2.00% | 14.00 |
| Strawberry flavouring | 8.00% | 56.00 |
| Allura red lake | 0.03% | 0.21 |
| Lemon yellow lake | 0.03% | 0.21 |
| Tablet weight | 100% | 700 |

According to the prescription in the table, EGCG was mixed with other ingredients, passed through a 40-mesh sieve, and pressed into tablets.

### Example 16

| Ingredient | Prescription 1 | | Prescription 2 | | Prescription 3 | |
|---|---|---|---|---|---|---|
| | Proportio n % | Prescription dose/mg | Proportio n % | Prescription dose/mg | Proportio n % | Prescription dose/mg |
| EGCG | 1.43% | 10.00 | 1.43% | 10.00 | 1.43% | 10.00 |
| Sorbitol | 80.17% | 561.19 | 78.67% | 550.69 | 77.17% | 540.19 |
| Fructooligosacc harides | 5.00% | 35.00 | 5.00% | 35.00 | 5.00% | 35.00 |
| Sucralose | 0.50% | 3.50 | 1.00% | 7.00 | 1.50% | 10.50 |
| Menthol | 0.30% | 2.10 | 0.30% | 2.10 | 0.30% | 2.10 |
| DL-malic acid | 0.50% | 3.50 | 0.50% | 3.50 | 0.50% | 3.50 |
| Aspartam e | 2.00% | 14.00 | 2.00% | 14.00 | 2.00% | 14.00 |
| Magnesiu m stearate | 2.00% | 14.00 | 2.00% | 14.00 | 2.00% | 14.00 |
| Strawberr y flavouring | 8.00% | 56.00 | 9.00% | 63.00 | 10.00% | 70.00 |
| Allura red lake | 0.09% | 0.63 | 0.09% | 0.63 | 0.09% | 0.63 |
| Lemon yellow lake | 0.01% | 0.07 | 0.01% | 0.07 | 0.01% | 0.07 |
| Tablet weight | 100% | 700.00 | 100% | 700.00 | 100% | 700.00 |

According to the prescription in the table, EGCG was mixed with other ingredients, passed through a 40-mesh sieve, and pressed into tablets.

### Example 17

Strawberry flavored buccal tablets, 300 tablets, tablet weight was 1200mg, the punching die was an oval punching die, 21*8mm.

| Ingredient | Proportion, % | Prescription dose/mg | 300 tablets/g |
|---|---|---|---|
| Sorbitol | 60.48% | 725.72 | 217.716 |
| Strawberry fruit powder | 15.00% | 180 | 54 |
| Skimmed milk powder | 15.00% | 180 | 54 |
| Fructo-oligosaccharides | 5.00% | 60 | 18 |
| EGCG | 0.83% | 10 | 3 |
| Magnesium stearate | 2.00% | 24 | 7.2 |
| Aspartame | 0.20% | 2.4 | 0.72 |
| Sucralose | 0.50% | 6 | 1.8 |
| DL-malic acid | 0.80% | 9.6 | 2.88 |
| Menthol | 0.10% | 1.2 | 0.36 |
| Allura red lake | 0.09% | 1.08 | 0.324 |
| Tablet weight | 100% | 1200 | 360 |

According to the prescription in the table, EGCG was mixed with other ingredients, passed through a 40-mesh sieve twice, mixed for 10 minutes, and pressed into tablets. The tablets had a hardness of about 200N.

### Example 18

| Ingredient | Proportion, % | Prescription dose/mg | 300 tablets/g |
|---|---|---|---|
| Sorbitol | 49.38% | 592.56 | 177.768 |
| Strawberry fruit powder/lemon fruit powder | 20.00% | 240 | 72 |
| Skimmed milk powder | 20.00% | 240 | 72 |
| Fructo-oligosaccharides | 5.00% | 60 | 18 |
| EGCG | 0.83% | 9.96 | 2.988 |
| Magnesium stearate | 3.00% | 36 | 10.8 |
| Aspartame | 0.20% | 2.4 | 0.72 |
| Sucralose | 0.60% | 7.2 | 2.16 |
| DL-malic acid | 0.80% | 9.6 | 2.88 |
| Menthol | 0.10% | 1.2 | 0.36 |
| Allura red aluminum lake/lemon yellow aluminum lack | 0.09% | 1.08 | 0.324 |
| Tablet weight | 100% | 1200 | 360 |

According to the prescription in the table, EGCG was mixed with other ingredients, passed through a 40-mesh sieve twice, mixed for 10 minutes, and pressed into tablets by a circular punch of 15mm. The tablets had a hardness of about 200N.

### Example 19

| Ingredient | Proportion, % | Prescription dose/mg |
|---|---|---|
| Sorbitol | 74.30% | 520.07 |
| Strawberry fruit powder | 9.00% | 63 |
| Skimmed milk powder | 8.00% | 56 |
| Fructo-oligosaccharides | 5.00% | 35 |
| Magnesium stearate | 2.00% | 14 |
| EGCG | 0.71% | 5 |
| Sucralose | 0.25% | 1.75 |
| DL-malic acid | 0.50% | 3.5 |
| Aspartame | 0.10% | 0.7 |
| Menthol | 0.05% | 0.35 |
| Allura red aluminum lake | 0.09% | 0.63 |
| Tablet weight | 100% | 700 |

According to the prescription in the table, EGCG was mixed with other ingredients, passed through a 40-mesh sieve, and pressed into tablets.

### Example 20

| Ingredient | Proportion, % | Prescription dose/mg |
|---|---|---|
| Sorbitol | 74.30% | 520.07 |
| Lemon fruit powder | 9.00% | 63 |
| Skimmed milk powder | 8.00% | 56 |
| Fructo-oligosaccharides | 5.00% | 35 |
| Magnesium stearate | 2.00% | 14 |
| EGCG | 0.71% | 5 |
| Sucralose | 0.25% | 1.75 |
| DL-malic acid | 0.50% | 3.5 |
| Aspartame | 0.10% | 0.7 |
| Menthol | 0.05% | 0.35 |
| Lemon yellow aluminum lake | 0.09% | 0.63 |
| Tablet weight | 100% | 700 |

According to the prescription in the table, EGCG was mixed with other ingredients, passed through a 40-mesh sieve, and pressed into tablets.

### Example 21

| Ingredient | Prescription 1 | | Prescription 2 | |
|---|---|---|---|---|
| | Proportion/% | Prescription dose/mg | Proportion/% | Prescription dose/mg |
| Sorbitol | 77.89% | 545.23 | 72.89% | 510.23 |
| Strawberry fruit powder | 9.00% | 63 | 9.00% | 63 |
| Skimmed milk powder | 8.00% | 56 | 8.00% | 56 |
| Fructo-oligosaccharides | / | / | 5.00% | 35 |
| Magnesium stearate | 2.00% | 14 | 2.00% | 14 |
| Aspartame | 1.00% | 7 | 1.00% | 7 |
| EGCG | 0.71% | 4.97 | 0.71% | 4.97 |
| Sucralose | 0.50% | 3.5 | 0.50% | 3.5 |
| DL-malic acid | 0.50% | 3.5 | 0.50% | 3.5 |
| Menthol | 0.30% | 2.1 | 0.30% | 2.1 |
| Allura red lake | 0.09% | 0.63 | 0.09% | 0.63 |
| Lemon yellow lake | 0.01% | 0.07 | 0.01% | 0.07 |
| Tablet weight | 100% | 700 | 100% | 700 |

According to the prescription in the table, EGCG was mixed with other ingredients, passed through a 40-mesh sieve, and pressed into tablets.

### Experimental Example 1: Determination of dissolution rate

The buccal tablets of Examples 6, 7 and 8 were taken and determined for the dissolution. The dissolution results were shown in Table 1 and Table 2. The dissolution rate was determined according to the method for determining dissolution rate (General Chapter 0931, Method 2, Chinese Pharmacopoeia, 2020 Edition), in which the medium was purified water, the temperature was 37.0°C, the volume was 900ml, and the time was 5 minutes, 10 minutes, 15 minutes, 20 minutes, and 30 minutes, respectively, and the rotation speed was 100rpm, 150rpm, respectively, the determination was performed through UV detector with detection wavelength of 207nm.

**Table 1**

| Dissolution conditions: paddle method, 100rpm, water medium, 37°C | | | |
|---|---|---|---|
| Time/minute | Example 6 | Example 7 | Example 8 |
| 5 | 60.5% | 54.8% | 57.8% |
| 10 | 94.6% | 93.1% | 91.3% |
| 15 | 99.7% | 101.4% | 98.0% |
| 20 | 99.7% | 102.6% | 97.8% |
| 30 | 100.6% | 103.6% | 99.0% |

**Table 2**

| Dissolution conditions: paddle method, 150rpm, water medium, 37°C | | | |
|---|---|---|---|
| Time/minute | Example 6 | Example 7 | Example 8 |
| 5 | 68.9% | 64.5% | 68.7% |
| 10 | 100.4% | 100.1% | 99.8% |
| 15 | 102.0% | 101.7% | 101.1% |
| 20 | 102.8% | 103.5% | 101.5% |
| 30 | 103.4% | 103.4% | 102.0% |

The results showed that the buccal tablets of Examples 6, 7 and 8 showed rapid dissolution at different dissolution rotation speeds, and could be completely dissolved and reach the dissolution plateau within 10 minutes.

The buccal tablets of Example 12 were taken and determined for the dissolution. The dissolution results were shown in Table 3. The dissolution rate was determined according to the method for determining dissolution rate (General Chapter 0931, Method 2, Chinese Pharmacopoeia, 2020 Edition), in which the medium was purified water, the temperature was 37.0°C, the volume was 900ml, and the time was 5 minutes, 10 minutes, 15 minutes, 20 minutes, and 30 minutes, respectively, and the rotation speed was 100rpm, the determination was performed through UV detector with detection wavelength of 207nm.

**Table 3**

| Time/minute | Hardness of 150N | Hardness of 225N |
|---|---|---|
| 5 | 26.4% | 22.7% |
| 10 | 73.5% | 73.4% |
| 15 | 92.8% | 94.6% |
| 20 | 98.7% | 103.7% |
| 30 | 98.7% | 104.8% |

The buccal tablets of Example 13 were taken and determined for the dissolution. The dissolution results were shown in Table 4. The dissolution rate was determined according to the method for determining dissolution rate (General Chapter 0931, Method 2, Chinese Pharmacopoeia, 2020 Edition), in which the medium was purified water, the temperature was 37.0°C, the volume was 900ml, and the time was 1 minutes, 3 minutes, 5 minutes, 10 minutes, 15 minutes, 20 minutes, and 30 minutes, respectively, and the rotation speed was 100rpm, the determination was performed through UV detector with detection wavelength of 207nm. The buccal tablets were chopped into particles (simulating chewing in buccal cavity) and then determined. Test results showed that when the buccal tablets were chopped into particles, 70.1% EGCG, i.e., 3.5 mg, could be dissolved in 1 minute. It could be seen that buccal tablets could quickly release EGCG after being chewed, the release concentration (15.28mM) of which could reach an effective concentration for inactivation of viruses in buccal cavity.

**Table 4**

| Time/minute | Buccal tablets being chopped into particles |
|---|---|
| 1 | 70.1% |
| 3 | 97.2% |
| 5 | 99.0% |
| 10 | 99.9% |
| 15 | 100.5% |
| 20 | 101.3% |
| 30 | 100.4% |

### Experimental Example 2: Taste evaluation

The buccal tablets of Examples 7 to 12 and 14 were taken, tasted, evaluated and scored. The results were shown in Table 5. The scoring criteria for each score were as follows (the average score adopted the counting and retention method with rounding to five):
5 points: Rich and harmonious aroma, sweet and sour taste, no bitter taste
4 points: Slightly light aroma, no peculiar smell, good taste, no bitterness
3 points: Light aroma, little miscellaneous taste, and generally bitter taste
2 points: No aroma, slightly pungent odor, poor taste, and heavy bitterness.
1 point: No aroma, pungent smell, extremely bitter and difficult to swallow.

**Table 5**

| Taste | 5 points | 4 points | 3 points | 2 points | 1 point | Average score |
|---|---|---|---|---|---|---|
| Example 7 | 0 people | 2 people | 13 people | 5 people | 0 people | 2.8 |
| Example 8 | 0 people | 4 people | 14 people | 2 people | 0 people | 3.1 |
| Example 9 | 1 people | 4 people | 14 people | 1 people | 0 people | 3.2 |
| Example 10 | 0 people | 4 people | 15 people | 1 people | 0 people | 3.2 |
| Example 11 | 0 people | 5 people | 14 people | 1 people | 0 people | 3.2 |
| Example 12 | 0 people | 0 people | 14 people | 6 people | 0 people | 2.7 |
| Example 14, Prescription 1 | 0 people | 1 people | 15 people | 4 people | 0 people | 2.8 |
| Example 14, Prescription 2 | 0 people | 8 people | 12 people | 0 people | 0 people | 3.4 |
| Example 14, Prescription 3 | 3 people | 13 people | 4 people | 0 people | 0 people | 4.0 |
| Example 14, Prescription 4 | 11 people | 9 people | 0 people | 0 people | 0 people | 4.6 |

### Experimental Example 3: Determination of complete dissolution time in buccal cavity

The buccal tablets of Example 15 was taken and tested with the assistance of 6 subjects. The average dissolution time of the buccal tablets of Example 15 in the buccal cavity of 6 subjects was 4 minutes and 19 seconds (the gender ratio of the subjects was 1:1). The specific results were shown in Table 6. The disintegration time of this batch of buccal tablets in the disintegration instrument was 8 minutes and 44 seconds; the average chewing and dissolution time in the buccal cavity was 17.7 seconds. It could be seen that the buccal tablets could release enough EGCG within 1-5 minutes to reach effective concentration to inactivate viruses in buccal cavity.

**Table 6**

| Subject | Complete dissolution time in buccal cavity | Complete dissolution time in buccal cavity (chewing) |
|---|---|---|
| WLX | 4 minutes 20 seconds | 17 seconds |
| QHZ | 3 minutes 45 seconds | 18 seconds |
| ZP | 4 minutes 10 seconds | 20 seconds |
| YL | 3 minutes 50 seconds | 16 seconds |
| LMY | 4 minutes 48 seconds | 17 seconds |
| DZQ | 5 minutes 02 seconds | 18 seconds |

### Experimental Example 4: Determination of powder properties

The powder properties of the mixed powder after being passed through a 40-mesh sieve before being pressed into tablets in Examples 7 to 12 and 14 were determined, and the results were shown in Table 7.

**Table 7**

| | Angle of repose (°) | Bulk density (g/mL) | Tap density (g/mL) | compressibility coefficient (%) |
|---|---|---|---|---|
| Example 7 | 41.10 | 0.45 | 0.58 | 22.41 |
| Example 8 | 41.33 | 0.44 | 0.58 | 24.14 |
| Example 9 | 41.56 | 0.44 | 0.59 | 25.42 |
| Example 10 | 41.77 | 0.45 | 0.58 | 22.41 |
| Example 11 | 42.23 | 0.45 | 0.58 | 22.41 |
| Example 12 | 43.53 | 0.39 | 0.52 | 25.00 |
| Example 14, Prescription 1 | 41.35 | 0.47 | 0.61 | 22.95 |
| Example 14, Prescription 2 | 40.67 | 0.46 | 0.59 | 22.03 |
| Example 14, Prescription 3 | 39.55 | 0.45 | 0.56 | 19.64 |
| Example 14, Prescription 4 | 38.42 | 0.43 | 0.52 | 17.31 |

| | | | | |
|---|---|---|---|---|
| Note: compressibility coefficient = (tap density - bulk density) / tap density * 100% | | | | |

It is generally believed that if the repose angle is ≤30°, the fluidity is good; if the repose angle is ≤40°, it can meet the fluidity needs in the production process; if the repose angle is ≥40°, the fluidity is poor, and measures need to be taken to ensure accurate dosage.

The compressibility coefficient reflects the difficulty of powder compaction, that is, the strength of the force between particles. For powders with good fluidity, the force between particles is relatively weak, and the values of bulk density and tap density are very close. For powders with poor fluidity, the force between particles is generally larger, and the bridge bonds between particles make that the value of bulk density is far lower than that of tap density.

For the four prescriptions in Example 14, the higher the proportion of fructo-oligosaccharides, the smaller the corresponding angle of repose, the better the powder fluidity, and the better the compaction performance.

### Experimental Example 5: Stability test

According to the relevant stability investigation item requirements under the guiding principles for stability testing of raw materials and preparations (Chinese Pharmacopoeia, 2020 Edition, Part Four, General Chapter 9001), the buccal tablets of Prescription 1 and Prescription 2 of Example 21 were investigated. The main investigation items included influencing factor test and accelerated test. The main investigation indicators of the influencing factor test included: characters, related substances (including impurity ECG (epicatechin gallate), other single impurities and total impurities), moisture, hardness, and disintegration time. The results were shown in Tables I to VI. The main investigation indicators of the accelerated test include characters, related substances (including impurity ECG (epicatechin gallate), other single impurities and total impurities), moisture, hardness, and disintegration time. The results were shown in Table VII and Table VIII.

**Table I: Influencing factor test - high temperature of 60°C**

| Sample No. | Investigation item | | Before loading sample | Time (day) | | |
|---|---|---|---|---|---|---|
| | | | | 5 | 10 | 30 |
| Prescription 1 (EGCG buccal tablets not containing fructo-oligosaccharides ) | Character | | Pink oval tablets | Red oval tablets | Red oval tablets | Reddish brown oval tablets |
| | Relevant substance | Impurity ECG | 0.68% | 0.69% | 0.71% | 0.64% |
| | | Other single impurities | 1.0% | 1.3% | 1.9% | 4.4% |
| | | Total impurities | 2.4% | 6.0% | 10.2% | 24.4% |
| | Moisture | | 1.2% | 0.89% | 0.77% | 0.88% |
| | Hardness | | 206.85 | 189.38 | 173.73 | 115.34 |
| | Disintegration time | | 11min16s to 13min 06s | 8min 40s to 11min 20s | 9min 48s to 10min 45s | 5min 20s to 7min 31s |

**Table II: Influencing factor test - high temperature of 60°C**

| Sample No. | Investigation item | | Before loading sample | Time (day) | | |
|---|---|---|---|---|---|---|
| | | | | 5 | 10 | 30 |
| Prescriptio n 2 (EGCG buccal tablets) | Character | | Pink oval tablets | Red oval tablets | Red oval tablets | Reddish brown oval tablets |
| | Relevant substance | Impurity ECG | 0.68% | 0.69% | 0.71% | 0.68% |
| | | Other single impurities | 0.91% | 1.0% | 1.2% | 1.7% |
| | | Total impurities | 2.6% | 5.0% | 9.0% | 12.2% |
| | Moisture | | 1.5% | 1.3% | 0.87% | 0.66% |
| | Hardness | | 205.12 | 195.73 | 186.61 | 153.21 |
| | | Disintegration time | 11min48s to 13min10s | 11min06s to 12min30s | 8min30s to 9min40s | 7min38s to 8min15s |

**Table III: Influencing factor test - high humidity of RH92.5%**

| Sample No. | Investigation item | | Before loading sample | Time (day) | | |
|---|---|---|---|---|---|---|
| | | | | 5 | 10 | 30 |
| Prescription 1 (EGCG buccal tablets not containing fructo-oligosaccharides) | Character | | Pink oval tablets | Pink oval tablets | Pink oval tablets | Pink oval tablets |
| | Relevant substance | Impurity ECG | 0.68% | 0.69% | 0.69% | 0.71% |
| | | Other single impurities | 1.0% | 0.94% | 1.0% | 1.0% |
| | | Total impurities | 2.4% | 2.8% | 3.1% | 2.9% |
| | Moisture | | 1.2% | 1.2% | 1.4% | 2.0% |
| | Hardness | | 206.85 | 193.08 | 162.67 | 100.34 |
| | Disintegration time | | 11min16s to 13min06s | 9min30s to 12min11s | 7min30s to 8 min30s | 4min23s to 5min12s |

**Table IV: Influencing factor test - high humidity of RH92.5%**

| Sample No. | Investigation item | | Before loading sample | Time (day) | | |
|---|---|---|---|---|---|---|
| | | | | 5 | 10 | 30 |
| Prescription 2 (EGCG buccal tablets) | Character | | Pink oval tablets | Pink oval tablets | Pink oval tablets | Pink oval tablets |
| | Relevant substance | Impurity ECG | 0.68% | 0.68% | 0.68% | 0.72% |
| | | Other single impurities | 0.91% | 0.94% | 0.98% | 0.91% |
| | | Total impurities | 2.6% | 2.5% | 2.8% | 2.8% |
| | Moisture | | 1.5% | 1.5% | 1.7% | 2.0% |
| | Hardness | | 205.12 | 202.73 | 197.22 | 178.34 |
| | Disintegration time | | 11min48s to 13min10s | 9min40s to 13min10s | 11min43s to 12min10s | 10min33s to 11min06s |

**Table V: Influencing factor test - light (4500lx±500lx)**

| Sample No. | Investigation item | | Before loading sample | Time (day) | | |
|---|---|---|---|---|---|---|
| | | | | 5 | 10 | 30 |
| Prescription 1 (EGCG buccal tablets not containing fructo-oligosaccharides) | Character | | Pink oval tablets | Pink oval tablets | Pink oval tablets | Pink oval tablets |
| | Relevant substance | Impurity ECG | 0.68% | 0.69% | 0.69% | 0.73% |
| | | Other single impurities | 1.0% | 0.96% | 0.90% | 0.75% |
| | | Total impurities | 2.4% | 2.6% | 2.7% | 2.3% |
| | Moisture | | 1.2% | 1.4% | 1.2% | 1.3% |
| | Hardness | | 206.85 | 203.63 | 201.29 | 175.32 |
| | | Disintegration time | 11min16s to 13min06s | 8min40s to 11min20s | 9min02s to 10min11s | 8min09s to 10min06s |

**Table VI: Influencing factor test - light (4500lx±500lx)**

| Sample No. | Investigation item | | Before loading sample | Time (day) | | |
|---|---|---|---|---|---|---|
| | | | | 5 | 10 | 30 |
| Prescription 2 (EGCG buccal tablets) | Character | | Pink oval tablets | Pink oval tablets | Pink oval tablets | Pink oval tablets |
| | Relevant substance | Impurity ECG | 0.68% | 0.68% | 0.68% | 0.73% |
| | | Other single impurities | 0.91% | 0.84% | 0.79% | 0.67% |
| | | Total impurities | 2.6% | 2.5% | 2.8% | 2.2% |
| | Moisture | | 1.5% | 1.4% | 1.5% | 1.5% |
| | Hardness | | 205.12 | 192.99 | 192.96 | 181.25 |
| | Disintegration time | | 11min48s to 13min10s | 10min10s to 12min40s | 9min08s to 11min30s | 8min58s to 10min45s |

**Table VII: Accelerated test - (40°C±2°C, RH75%±5%)**

| Sample No. | Investigation item | | Before loading sample | Time (month) | |
|---|---|---|---|---|---|
| | | | | 1 | 2 |
| Prescription 1 (EGCG buccal tablets not containing fructo-oligosaccharides) | Character | | Pink oval tablets | Pink oval tablets | Red oval tablets |
| | Relevant substance | Impurity ECG | 0.68% | 0.76% | 0.69% |
| | | Other single impurities | 1.0% | 0.93% | 1.2% |
| | | Total impurities | 2.4% | 4.2% | 5.1% |
| | Moisture | | 1.2% | 1.7% | 2.8% |
| | Hardness | | 206.85 | 163.73 | 125.34 |
| | Disintegration time | | 11min16s to 13min06s | 8min58s to 9min45s | 6min11s to 7min25s |

**Table VIII: Accelerated test - (40°C±2°C, RH75%±5%)**

| Sample No. | Investigation item | | Before loading sample | Time (month) | |
|---|---|---|---|---|---|
| | | | | 1 | 2 |
| Prescription 2 (EGCG buccal tablets) | Character | | Pink oval tablets | Red oval tablets | Red oval tablets |
| | Relevant substance | Impurity ECG | 0.68% | 0.81% | 0.67% |
| | | Other single impurities | 0.91% | 1.3% | 1.0% |
| | | Total impurities | 2.6% | 4.5% | 4.9% |
| | Moisture | | 1.5% | 3.9% | 3.5% |
| | Hardness | | 205.12 | 173.68 | 158.34 |
| | Disintegration time | | 11min48s to 13min10s | 9min18s to 10min21s | 8min18s to |
| | | | | | 9min32s |

The stability of EGCG is affected by many factors, such as light, oxygen, pH value and ionic strength. In this experimental example, the stability of the buccal tablets of the present application was investigated, and it was found that adding fructo-oligosaccharides to the prescriptions could effectively improve the stability of EGCG. The stability test showed that fructo-oligosaccharides had more advantages in improving the stability of EGCG, the stability of EGCG was improved.

Experimental Example 6: In vitro evaluation of effect of EGCG on SARS-CoV-2 BJ-01 virus infection

In this experimental example, SARS-CoV-2 BJ-01 virus was provided by the Military Veterinary Research Institute, Academy of Military Medical Sciences, Academy of Military Sciences, and its Accession Number at NCBI was MT291831; Vero-E6 cells were provided by the Military Veterinary Research Institute, Academy of Military Medical Sciences, Academy of Military Sciences; and the culture medium was Dulbecco's Modified Eagle Medium, purchased from Thermo Scientific, catalog number: C11995500BT.

The incubation of drug EGCG with SARS-CoV-2 BJ-01 virus in the culture medium was designed according to the time gradient, in which the incubation time was: 1 minute, 2 minutes, 4 minutes, 8 minutes, the EGCG concentration in the incubation system was 1 mg/mL (2.18 mM), and the virus titer in the incubation system was 10⁷ TCID₅₀. After incubation, the incubation system was diluted 10 times and used to infect Vero-E6 cells. After 48 hours, the Ct value of the cell supernatant was detected. The experimental results were shown in Figure 1. After the co-incubation of SARS-CoV-2 BJ-01 at known titers with 2.18 mM EGCG at room temperature for 1 minute, 2 minutes, 4 minutes and 8 minutes, the number of live viruses was significantly reduced, suggesting that 2.18 mM EGCG could quickly and effectively inactivate SARS-CoV-2 within 1 minute.

Experimental Example 7: Evaluation of EGCG affecting on SARS-CoV-2 Omicron BA.2 infection and replication in model mice

In this experimental example, SARS-CoV-2 Omicron BA.2 was provided by the Military Veterinary Research Institute, Academy of Military Medical Sciences, Academy of Military Sciences; BALB/c mice were purchased from Beijing Vital River Laboratory Animal Technology Co., Ltd.; the culture medium was Dulbecco's Modified Eagle Medium, purchased from Thermo Scientific, catalog number: C11995500BT.

In the test of this experimental example, EGCG and the virus were first mixed and co-incubated in vitro to simulate the rapid inactivation of virus by EGCG in the buccal cavity. Then the mice were intranasally inoculated with a co-incubation solution of EGCG and the virus, and the replication of the virus quickly inactivated by EGCG in the mice was evaluated.

BALB/c mice (6 to 8 months old) were used as animal models. The experimental mice were randomly divided into 3 groups, i.e., the blank group (inoculated intranasally with 50 µL of culture medium without virus), the BA.2 virus control group (inoculated intranasally with 50 µL of culture medium containing virus), and the EGCG and virus co-incubation group (inoculated intranasally with 50 µL of a co-incubation solution produced by incubating 6.9mg of EGCG dissolved in 1mL of virus-containing culture medium (with virus titer of 10⁴ TCID₅₀) for 10 minutes, in which the EGCG concentration was 6.9 mg/mL, i.e., 15.06mM), 6 mice in each group. The virus concentration in the virus-containing culture medium involved in each group was the same, and the dose of virus inoculated to the mice of each group was 0.5×10³ TCTD₅₀ (Omicron BA.2 strain). The mice were dissected on 3^{rd} day and 5^{th} day after the virus challenge and infection, 2 mice in each group were dissected, and the turbinates and lung tissues of the mice were taken to determine the virus titer and viral load.

The experimental results were shown in Figure 2 and Tables A and B. As compared with the virus control group, when EGCG was co-incubated with the virus for 10 minutes, the viral loads of SARS-CoV-2 Omicron BA.2 were significantly reduced on 3^{rd} day (A and B) and 5^{th} day (C and D) after the virus challenge in the lung (on 3^{rd} day, the average Log value decreased by 2.07, and on 5^{th} day, the average Log value decreased by 1.30) and the turbinate tissues (on 3^{rd} day, the average Log value decreased by 2.51, and on 5^{th} day, the average Log value decreased by 2.98) of the mice. It could be seen that EGCG could quickly inactivate viruses in the buccal cavity and throat. Even if the inactivated virus was absorbed by mice, its replication and proliferation in vivo tissues could be significantly inhibited.

**Table A: Viral load (number of viral gene copies) in lungs and turbinates of mice in different groups**

| | Group | Virus control group | | Co-incubation group | |
|---|---|---|---|---|---|
| | Mouse No./Tissue | Lung | Turbinate | Lung | Turbinate |
| Viral genome copy number on 3^{rd} day after challenge | 1 | 288019.8229 | 896335.6783 | 1515.779958 | 2784.622024 |
| | 2 | 235074.5963 | 1464285.148 | 3180.04242 | 4535.536869 |
| Viral genome copy number on 5^{th} day after challenge | 3 | 22235.22734 | 329339.041 | 204.1526212 | 176.7425718 |
| | 4 | 29461.57635 | 144161.569 | 3231.818414 | 346.8978229 |
| | 5 | 89297.83527 | 268008.0085 | 2001.950579 | 78.66951809 |
| | 6 | 38815.21594 | 1043089.792 | 10432.24969 | 3377.924226 |

**Table B: Viral load in the lungs and turbinates of mice in different groups (Log₁₀ value of viral gene copy number)**

| | Group | Virus control group | | Co-incubation group | |
|---|---|---|---|---|---|
| | Mouse No./Tissue | Lung | Turbinate | Lung | Turbinate |
| Viral genome copy number on 3^{rd} day after challenge | 1 | 5.46 | 5.95 | 3.18 | 3.44 |
| | 2 | 5.37 | 6.17 | 3.50 | 3.66 |
| Viral genome copy number on 5^{th} day after challenge | 3 | 4.35 | 5.52 | 2.31 | 2.25 |
| | 4 | 4.47 | 5.16 | 3.51 | 2.54 |
| | 5 | 4.95 | 5.43 | 3.30 | 1.90 |
| | 6 | 4.59 | 6.02 | 4.02 | 3.53 |

Experimental Example 8: Evaluation of EGCG affecting on SARS-CoV-2/C57MA14 infection and replication in model mice

In this experimental example, SARS-CoV-2/C57MA14 was provided by the Institute of Military Veterinary Medicine, Academy of Military Medical Sciences, Academy of Military Sciences, which was is the mouse-adapted strain of clinically isolated SARS-CoV-2 successfully obtained by continuous passage in C57BL/6N mice; BALB/c mice were purchased from Beijing Vital River Laboratory Animal Technology Co., Ltd.; the culture medium was Dulbecco's Modified Eagle Medium, purchased from Thermo Scientific, catalog number: C11995500BT.

In the test of this experimental example, EGCG and the virus were first mixed and incubated in vitro to simulate the rapid inactivation of the virus by EGCG in the buccal cavity. Then the mice were intranasally inoculated with a co-incubation solution of EGCG and the virus, and the replication of the virus quickly inactivated by EGCG in the mice was evaluated.

BALB/c mice (6 to 8 months old) were used as animal models. The experimental mice were randomly divided into 3 groups, i.e., the blank group (inoculated intranasally with 50 µL of culture medium without virus), the SARS-CoV-2/C57MA14 virus control group (inoculated intranasally with 50 µL of culture medium containing virus), the EGCG and SARS-CoV-2/C57MA14 virus co-incubation group (inoculated intranasally with 50 µL of a co-incubation solution produced by incubating 6.9 mg of EGCG dissolved in 1 mL of virus-containing culture medium (with virus titer of 10⁷ TCID₅₀) for 10 minutes, in which the EGCG concentration was 6.9 mg/mL, i.e., 15.06mM), 6 animals in each group. The virus concentration in the virus-containing culture medium involved in each group was the same, and the dose of virus inoculated to the mice in each group was 1× LD₅₀ (SARS-CoV-2/C57MA14 strain). The mice were dissected on the 3^{rd} day and 5^{th} day after virus challenge and infection, 2 mice were dissected in each group, and the corresponding indicators in each group were determined to evaluate the drug effect, in which: ① indicator 1: the survival status of each group was monitored; ② indicator 2: the turbinates and lung tissues of mice were taken to determine the virus titer and viral load.

The experimental results were shown in Figure 3, Table C and Table D. Compared with the virus control group, the co-incubation of EGCG and virus for 10 minutes could not only protected 60% of mice from death caused by lethal dose SARS-CoV-2 C57MA14 infection (A), but also significantly reduce the viral loads of the lungs (the average Log value decreased by 5.56 on 3^{rd} day, and the average Log value decreased by 4.98 on 5^{th} day) and the turbinate tissues (the average Log value decreased by 4.18 on 3^{rd} day, and the average Log value decreased by 4.12 on 5^{th} day) in the mice on 3^{rd} day (B and C) and 5^{th} day (D and E) after virus challenge. It was suggested that EGCG could quickly inactivate SARS-CoV-2 C57MA14 in the buccal cavity and throat. Even if the inactivated virus was absorbed by the mice, its replication and proliferation in vivo tissues could be significantly inhibited.

**Table C: Viral load (number of viral gene copies) in lungs and turbinates of mice in different groups**

| | Group | Virus control group | | Co-incubation group | |
|---|---|---|---|---|---|
| | Mouse No./Tissue | Lung | Turbinate | Lung | Turbinate |
| Viral genome copy number on 4rd day after challenge | 1 | 189823607.3 | 3166439.342 | 10545.86063 | 1192.125616 |
| | 2 | 489151276.8 | 13927992.28 | 1335.47907 | 912.9740802 |
| Viral genome copy number on 5^{th} day after challenge | 3 | 104826466.7 | 6060827.546 | 1088.941369 | 1806.407862 |
| | 4 | 123662255.9 | 2324270.467 | 975.9276057 | 172.9855142 |
| | 5 | | | 1615.862245 | 76.49262114 |

**Table D: Virus load in lungs and turbinates of mice in different groups (Log value of viral gene copy number)**

| | Group | Virus control group | | Co-incubation group | |
|---|---|---|---|---|---|
| | Mouse No./Tissue | Lung | Turbinate | Lung | Turbinate |
| Viral genome copy number on 3^{rd} day after challenge | 1 | 8.28 | 6.50 | 4.02 | 3.08 |
| | 2 | 8.69 | 7.14 | 3.13 | 2.96 |
| Viral genome copy number on | 3 | 8.02 | 6.78 | 3.04 | 3.26 |
| | 4 | 8.09 | 6.37 | 2.99 | 2.24 |
| 5^{th} day after challenge | 5 | | | 3.21 | 1.88 |

### Experimental Example 9: Determination of inactivation efficiency of EGCG against influenza A H1N1-PR8 virus strain

### (1) Experimental materials

1. Epigallocatechin gallate (EGCG): purchased from Guangzhou Jiuzhi Pharmaceutical Technology Co., Ltd., catalog number: PM-0017;
2. Madin-darby canine kidney cells (MDCK): purchased from American Type Culture Collection (ATCC);
3. Human rhabdomyosarcoma cells (RD): purchased from American Type Culture Collection (ATCC), ATCC Number: CCL-136;
4. Liver cancer cells (Huh-7): purchased from American Type Culture Collection (ATCC);
5. Influenza A H1N1 virus (H1N1-PR8): purchased from American Type Culture Collection (ATCC);
6. Influenza A H3N2 virus (H3N2): purchased from American Type Culture Collection (ATCC);
7. Influenza B virus (B/Lee): purchased from American Type Culture Collection (ATCC), ATCC Number: VR-1535;
8. Influenza A virus neuraminidase inhibitor-resistant strain (ZX-1109): purchased from American Type Culture Collection (ATCC);
9. Novel enterovirus D68 (EV-D68): purchased from American Type Culture Collection (ATCC);
10. Coronavirus (hCoV-229E): purchased from American Type Culture Collection (ATCC), ATCC Number: VR-740;
11. DMEM culture medium: purchased from Gibco Company, catalog number: 11995065;
12. D/F-12 culture medium: purchased from Gibco Company, catalog number: 11330032;
13. FBS: purchased from Gibco Company, catalog number: 16000044;
14. TPCK-Trypsin: purchased from Sigma Company, catalog number: T1426;
15. DEPC water: purchased from Beijing Like Biotechnology Co., Ltd., catalog number: lk0710992
16. ddH₂O (RNase-free Water): purchased from Takara Company, catalog number: 9012
17. DPBS: purchased from Sigma Company, catalog number: D8537;
18. 0.25% Trypsin-EDTA: purchased from Gibco Company, catalog number: 25200072.
19. Culture bottle: 75cm² culture bottle, purchased from Corning Incorporated, catalog number: 430720;
20. Centrifuge tubes: 50mL centrifuge tubes, purchased from Corning Incorporated, catalog number: 430828; 15mL centrifuge tubes, purchased from Corning Incorporated, catalog number: 430790; 1.5mL centrifuge tubes, purchased from Axygen Company, catalog number: MCT-150-C;
21. Pipette: 5mL pipette, purchased from Corning Incorporated, catalog number: 4487; 10mL pipette, purchased from Corning Incorporated, catalog number: 4488;
22. 96-well plate: purchased from Corning Incorporated, catalog number: 3599;
23. Pipettor: purchased from Eppendorf;
24. Pipetting tips: 10 µL tip, purchased from QSP Company, catalog number: T102RS-Q; 200 µL tip, purchased from QSP Company, catalog number: T090RS-Q; 1mL tip, purchased from Axygen Company, catalog number: TF-1000-R-S;
25. Reagent tank: purchased from Biologix Group Ltd., catalog number: 25-0051.

### (2) Experimental method:

### 1. Treatment of compound

According to the molecular weight and mass of EGCG, DEPC water was used to dissolve the compound into a solution with a concentration of 100mM. After the sample was completely dissolved, it was filtered and sterilized with a 0.22µm filter membrane for later use.

### 2. Evaluation of antiviral activity

1) MDCK cells (1.5×10⁴/well) were inoculated into a 96-well plate and cultured overnight.
2) Dilution of compound and action on virus.
   ① The final concentration of EGCG was 5 mM: 5 µL of the compound stock solution was added to 95 µL of virus solution, mixed and allowed to stand at room temperature for 1 minute or 5 minutes;
   ② The final concentration of EGCG was 1 mM: 10 µL of the compound stock solution was added to 40 µL of virus culture medium, mixed well, then 5 µL of the mixed solution was taken and added to 95 µL of virus solution, mixed and allowed to stand at room temperature for 1 minute or 5 minutes;
   ③ The final concentration of EGCG was 0.2 mM: 2 µL of the compound stock solution was added to 48 µL of virus culture medium, mixed well, then 5 µL of the mixed solution was taken and added to 95 µL of virus solution, mixed and allowed to stand at room temperature for 1 minute or 5 minutes;
   ④ The negative control group only contained the compound at corresponding concentrations and the virus culture medium, the positive control group only contained the corresponding solvent and virus, and other treatment conditions were the same as those of the experimental group.
3) The cell culture medium was discarded, washing was performed three times with 150 µL DPBS, and then 150 µL of virus culture medium was added to each well.
4) The mixed solution in 2) was diluted ten times in gradient, 50 µL of the dilution solution was add to each well to infect cells, 10 duplicate wells were set up for each dilution solution, and the residual virus titer (TCID₅₀) was determined.
5) After 72 hours of infection, the virus titer was calculated by Karber method.
6) The inactivation efficiency of the sample was calculated by the results of three repetition experiments.
(3) Experimental results

### 1. Inactivation efficiency of EGCG against influenza A virus H1N1-PR8 within action time of 1 minute

### 1) Results of the first independent repetition (Table 8):

For the negative control group, cytotoxicity was observed in 5mM group and 1mM group. The cells in the 0.2mM group were in good growth status, and there was no virus replication.

For the positive control group, the TCID₅₀/50µL of the ddH₂O+virus group was calculated by the Karber method and was 10^{6.6}.

For the experimental group, virus replication was observed in each experimental group, the TCID₅₀/50µL of the 5 mM EGCG+virus group was calculated by the Karber method and was 10^{4.3}, the TCID₅₀/50µL of the 1 mM EGCG+virus group was 10^{5.7}, and the TCID₅₀/50µL of the 0.2 mM EGCG+virus group was 10^{6.3}. According to the calculation by the formula, the inactivation efficiency of 5 mM EGCG against the virus was 99.50%, the inactivation efficiency of 1mM EGCG against the virus was 87.41%, and the inactivation efficiency of 0.2 mM EGCG against the virus was 49.88%.

**Table 8: Inactivation effect of EGCG at different concentrations against virus when co-incubated with PR8 virus for 1 minute (first experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | + | + |
| | 10⁷ | - | - | - | + | - | - | - | - | - | - |
| | 10⁸ | - | - | - | - | - | - | - | - | - | - |

| | | | | | | | | | | | |
|---|---|---|---|---|---|---|---|---|---|---|---|
| Group | Dilution factor | 1 | 2 | 3 | 4 | 5 | 6 | 7 | 8 | 9 | 10 |
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | + | + | + | - | + | + | - | - | + | + |
| | 10⁵ | - | - | + | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Group | Dilution factor | 1 | 2 | 3 | 4 | 5 | 6 | 7 | 8 | 9 | 10 |
| Experimental group (1mM EGCG+virus) | 10¹ | * | * | * | * | * | * | * | * | * | * |
| | 10² | * | * | * | * | * | * | * | * | * | * |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | - | - | - | - | - | - | + | + |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Group | Dilution factor | 1 | 2 | 3 | 4 | 5 | 6 | 7 | 8 | 9 | 10 |
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | + | + | - | + | + | + | + | - | + |
| | 10⁷ | + | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Group | Dilution factor | 1 | 2 | 3 | 4 | 5 | 6 | 7 | 8 | 9 | 10 |
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 2) Results of the second independent repetition (Table 9):

For the negative control group, cytotoxicity was observed in 5mM group and 1mM group. The cells in the 0.2mM group were in good growth status, and there was no virus replication.

For the positive control group, the TCID₅₀/50µL of the ddH₂O+virus group was calculated by the Karber method and was 10^{6.6}.

For the experimental group, virus replication was observed, the TCID₅₀/50µL of the 5 mM EGCG+virus group was calculated by the Karber method and was 10^{4.2}, the TCID₅₀/50µL of the 1 mM EGCG+virus group was 10^{5.9}, and the TCID₅₀/50µL of the 0.2 mM EGCG+virus group was 10^{6.3}. According to the calculation by the formula, the inactivation efficiency of 5 mM EGCG against the virus was 99.60%, the inactivation efficiency of 1mM EGCG against the virus was 80.05%, and the inactivation efficiency of 0.2 mM EGCG against the virus was 49.88%.

**Table 9: Inactivation effect of EGCG at different concentrations against virus when co-incubated with PR8 virus for 1 minute (second experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | + | + |
| | 10⁷ | - | - | - | + | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | + | - | - | + | + | + | | - | - | - |
| | 10⁵ | - | - | + | - | + | - | - | + | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | + | - | - | + | + | - | + | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2 mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | - | + | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 3) Results of the third independent repetition (Table 10):

For the negative control group, cytotoxicity was observed in 5mM group and 1mM group. The cells in the 0.2mM group were in good growth status, and there was no virus replication.

For the positive control group, the TCID₅₀/50µL, of the dd H₂O+virus group was calculated by the Karber method and was 10^{6.6}.

For the experimental group, virus replication was observed, the TCID₅₀/50µL of the 5 mM EGCG+virus group was calculated by the Karber method and was 10^{5.4}, the TCID₅₀/50µL of the 1 mM EGCG+virus group was 10^{5.9}, and the TCID₅₀/50µL of the 0.2 mM EGCG+virus group was 10^{6.3}. According to the calculation by the formula, the inactivation efficiency of 5 mM EGCG against the virus was 93.69%, the inactivation efficiency of 1mM EGCG against the virus was 80.05%, and the inactivation efficiency of 0.2 mM EGCG against the virus was 49.88%.

**Table 10: Inactivation effect of EGCG at different concentrations against virus when co-incubated with PR8 virus for 1 minute (third experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | + | + |
| | 10⁷ | - | - | - | + | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | + | + | + | + | + | + | + | + | + | + |
| | 10⁵ | + | - | + | + | + | + | + | + | + | - |
| | 10⁶ | - | - | + | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | * | * | * | * | * | * | * | * | * | * |
| | 10³ | + | + | + | + | + | + | + | + | + | + |
| | 10⁴ | + | + | + | + | + | + | + | + | + | + |
| | 10⁵ | + | + | + | + | + | - | + | + | + | + |
| | 10⁶ | + | - | + | + | + | - | - | - | - | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | + | - | - | + | + | + | + | + | + |
| | 10⁷ | - | - | + | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

4) Conclusion on action time of 1 minute: In this experiment, the results of three experiments were statistically analyzed and it was found that when 5mM EGCG was incubated with PR8 virus for 1 minute, the average inactivation efficiency against virus was 97.60%; when 1mM EGCG was incubated with PR8 virus for 1 minute, the average inactivation efficiency against virus was 82.50%; and when 0.2mM EGCG was incubated with PR8 virus for 1 minute, the average inactivation efficiency against virus was 49.88%. In summary, the EGCG treatment could effectively inactivate influenza A H1N1 virus PR8 strain.

### 2. Inactivation efficiency of EGCG against influenza A virus H1N1-PR8 strain within action time of 5 minutes

### 1) Results of the first independent repetition (Table 11):

For the negative control group, cytotoxicity was observed in 5mM group and 1mM group. The cells in the 0.2mM group were in good growth status, and there was no virus replication.

For the positive control group, the TCID₅₀/50µL of the dd H₂O+virus group was calculated by the Karber method and was 10^{6.6}.

For the experimental group, virus replication was observed in each experimental group, the TCID₅₀/50µL of the 5 mM EGCG+virus group was calculated by the Karber method and was 10^{3.5}, the TCID₅₀/50µL of the 1 mM EGCG+virus group was 10^{5.7}, and the TCID₅₀/50µL of the 0.2 mM EGCG+virus group was 10^{6.1}. According to the calculation by the formula, the inactivation efficiency of 5 mM EGCG against the virus was 99.92%, the inactivation efficiency of 1mM EGCG against the virus was 87.41%, and the inactivation efficiency of 0.2 mM EGCG against the virus was 68.38%.

**Table 11 Inactivation effect of EGCG at different concentrations against virus when co-incubated with PR8 virus for 5 minutes (first experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | + | + |
| | 10⁷ | - | - | - | + | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | * | * | * | * | * | * | * | * | * | * |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | + | - | - | - | - | - | - | - | - |
| | 10⁷ | - | - | + | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | - | - | + | + | - | + | - | + | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| Negative control group (1mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 2) Results of the second independent repetition (Table 12):

For the negative control group, cytotoxicity was observed in 5mM group and 1mM group. The cells in the 0.2mM group were in good growth status, and there was no virus replication.

For the positive control group, the the TCID₅₀/50µL of the dd H₂O+virus group was calculated by the Karber method and was 10^{6.6}.

For the experimental group, virus replication was observed in each experimental group, the TCID₅₀/50µL of the 5 mM EGCG+virus group was calculated by the Karber method and was 10⁴, the TCID₅₀/50µL of the 1 mM EGCG+virus group was 10^{5.8}, and the TCID₅₀/50µL of the 0.2 mM EGCG+virus group was 10^{6.2}. According to the calculation by the formula, the inactivation efficiency of 5 mM EGCG against the virus was 99.75%, the inactivation efficiency of 1mM EGCG against the virus was 84.15%, and the inactivation efficiency of 0.2 mM EGCG against the virus was 60.19%.

**Table 12 Inactivation effect of EGCG at different concentrations against virus when co-incubated with PR8 virus for 5 minutes (second experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | + | + |
| | 10⁷ | - | - | - | + | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | + | - | - | - | - | - | - | - | - | - |
| | 10⁵ | - | - | + | + | + | - | + | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | * | * | * | * | * | * | * | * | * | * |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | + | - | - | - | + | + | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | + | - | + | + | + | + | + | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 3) Results of the third independent repetition (Table 13):

For the negative control group, cytotoxicity was observed in 5mM group and 1mM group. The cells in the 0.2mM group were in good growth status, and there was no virus replication.

For the positive control group, the TCID₅₀/50µL of the dd H₂O+virus group was calculated by the Karber method and was 10^{6.6}.

For the experimental group, virus replication was observed in each experimental group, the TCID₅₀/50µL of the 5 mM EGCG+virus group was calculated by the Karber method and was 10^{4.2}, the TCID₅₀/50µL of the 1 mM EGCG+virus group was 10^{5.6}, and the TCID₅₀/50µL of the 0.2 mM EGCG+virus group was 10^{5.8}. According to the calculation by the formula, the inactivation efficiency of 5 mM EGCG against the virus was 99.60%, the inactivation efficiency of 1mM EGCG against the virus was 90.00%, and the inactivation efficiency of 0.2 mM EGCG against the virus was 84.15%.

**Table 13 Inactivation effect of EGCG at different concentrations on virus when co-incubated with PR8 virus for 5 minutes (third experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | + | + |
| | 10⁷ | - | - | - | + | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | + | + | - | - | - | + | - | - | + | + |
| | 10⁵ | + | - | - | - | - | + | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | * | * | * | * | * | * | * | * | * | * |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | - | - | + | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | - | - | - | - | - | + | - | - |
| | 10⁷ | - | - | - | - | - | - | - | - | - | - |
| | 10⁸ | - | - | - | - | - | - | - | - | - | - |
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

4) Conclusion on action time of 5 minutes: In this experiment, the results of three experiments were statistically analyzed and it was found that when 5mM EGCG was incubated with PR8 virus for 5 minutes, the average inactivation efficiency against virus was 99.76%; when 1mM EGCG was incubated with PR8 virus for 5 minutes, the average inactivation efficiency against virus was 87.19%; and when 0.2mM EGCG was incubated with PR8 virus for 5 minutes, the average inactivation efficiency against virus was 70.91%. In summary, the EGCG treatment could effectively inactivate influenza A H1N1 virus PR8 strain.

### Experimental Example 10: Determination of inactivation efficiency of EGCG against influenza A H3N2 virus

### (1) Experimental materials

Same as Experimental Example 9.

### (2) Experimental method:

### 1. Treatment of compound

According to the molecular weight and mass of EGCG, DEPC water was used to dissolve the compound into a solution with a concentration of 100mM. After the sample was completely dissolved, it was filtered and sterilized with a 0.22µm syringe filter for later use.

### 2. Evaluation of antiviral activity

1) MDCK cells (1.5 × 10⁴ cells/well) were inoculated into a 96-well plate and cultured overnight.
2) Dilution of compound and action on virus
   ① The final concentration of EGCG was 5 mM: 5 µL of the compound stock solution was added to 95 µL of the virus solution, mixed and allowed to stand at room temperature for 1 minute or 5 minutes;
   ② The final concentration of EGCG was 1 mM: 10 µL of the compound stock solution was added to 40 µL of virus culture medium, mixed well, then 5 µL of the mixed solution was taken and added to 95 µL of the virus solution, mixed and allowed to stand at room temperature for 1 minute or 5 minutes;
   ③ The final concentration of EGCG was 0.2 mM: 2 µL of the compound stock solution was added to 48 µL of virus culture medium, mixed well, then 5 µL of the mixed solution was taken and added to 95 µL of the virus solution, mixed and allowed to stand at room temperature for 1 minute or 5 minutes;
   ④ The negative control group only contained the compound at corresponding concentrations and the virus culture medium, the positive control group only contained the corresponding solvent and virus, and other treatment conditions were the same as those of the experimental group.
3) The cell culture medium was discarded, washing was performed three times with 150 µL DPBS, and then 150 µL of the virus culture medium was added to each well.
4) The mixed solution in 2) was diluted ten times in gradient, 50 µL of the dilution solution was add to each well to infect cells, 10 duplicate wells were set up for each dilution solution, and the residual virus titer (TCID₅₀) was determined.
5) After 72 hours of infection, the virus titer was calculated by Karber method.
6) The inactivation efficiency of the sample was calculated by the results of three repetition experiments.

### (3) Experimental results

### 1. Inactivation efficiency of EGCG against influenza A virus H3N2 within action time of 1 minute

### 1) Results of the first independent repetition (Table 14):

For the negative control group, cytotoxicity was observed in the 5mM group and in the sample of 10 times dilution in the 1mM group. The cells in other samples in the 1 mM group and the cells in the 0.2 mM group were in good growth status, and there was no virus replication.

For the positive control group, the TCID₅₀/50µL of the dd H₂O+virus group was calculated by the Karber method and was 10^{5.4}.

For the experimental group, virus replication was observed in each experimental group, the TCID₅₀/50µL of the 5 mM EGCG+virus group was calculated by the Karber method and was 10^{3.5}, the TCID₅₀/50µL of the 1 mM EGCG+virus group was 10^{4.9}, and the TCID₅₀/50µL of the 0.2 mM EGCG+virus group was 10^{5.2}. According to the calculation by the formula, the inactivation efficiency of 5 mM EGCG against the virus was 98.74%, the inactivation efficiency of 1mM EGCG against the virus was 68.38%, and the inactivation efficiency of 0.2 mM EGCG against the virus was 36.90%.

**Table 14 Inactivation effect of EGCG at different concentrations against virus when co-incubated with H3N2 virus for 1 minute (first experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | - | - | - | + | + | - | + | - | + |
| | 10⁶ | + | - | - | - | - | - | - | - | + | + |
| | 10⁷ | - | - | - | - | - | - | + | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | - | - | + | + | - | - | + | + | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | + | - | + | + | + | + | - | + | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 2) Results of the second independent repetition (Table 15):

For the negative control group, cytotoxicity was observed in the 5mM group and in the sample of 10 times dilution of in the 1mM group. The cells in other samples in the 1mM group and the cells in the 0.2mM group were in good growth status, and there was no virus replication.

For the positive control group, the TCID₅₀/50µL, of the dd H₂O+virus group was calculated by the Karber method and was 10^{5.4}.

For the experimental group, virus replication was observed in each experimental group, the TCID₅₀/50µL, of the 5 mM EGCG+virus group was calculated by the Karber method and was 10^{1.5}, the TCID₅₀/50µL of the 1 mM EGCG+virus group was 10^{4.7}, and the TCID₅₀/50µL of the 0.2 mM EGCG+virus group was 10^{5.1}. According to the calculation by the formula, the inactivation efficiency of 5 mM EGCG against the virus was 98.74%, the inactivation efficiency of 1mM EGCG against the virus was 80.05%, and the inactivation efficiency of 0.2 mM EGCG against the virus was 49.88%.

**Table 15 Inactivation effect of EGCG at different concentrations against virus when co-incubated with H3N2 virus for 1 minute (second experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | - | - | - | + | + | - | + | - | + |
| | 10⁶ | + | - | - | - | - | - | - | - | + | + |
| | 10⁷ | - | - | - | - | - | - | + | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | + | * | * | * | * |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | * | * | * | * | * | * | * | * | * |
| | 10² | + | + | * | * | + | + | + | * | * | * |
| | 10³ | + | + | + | + | + | + | + | + | + | + |
| | 10⁴ | - | + | + | + | + | + | + | + | + | + |
| | 10⁵ | + | - | - | - | - | - | - | - | + | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | - | - | + | + | + | + | - | + | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 3) Results of the third independent repetition (Table 16):

For the negative control group, cytotoxicity was observed in the 5mM group and in the sample of 10 times dilution in the 1mM group. The cells in other samples in the 1 mM group and the cells in the 0.2 mM group were in good growth status, and there was no virus replication.

For the positive control group, the TCID₅₀/50µL of the dd H₂O+virus group was calculated by the Karber method and was 10⁵.

For the experimental group, virus replication was observed in each experimental group, the TCID₅₀/50µL, of the 5 mM EGCG+virus group was calculated by the Karber method and was 10^{3.5}, the TCID₅₀/50µL of the 1 mM EGCG+virus group was 10^{4.8}, and the TCID₅₀/50µL of the 0.2 mM EGCG+virus group was 10⁵. According to the calculation by the formula, the inactivation efficiency of 5 mM EGCG against the virus was 96.84%, the inactivation efficiency of 1mM EGCG against the virus was 36.90%, and the inactivation efficiency of 0.2 mM EGCG against the virus was 0%.

**Table 16 Inactivation effect of EGCG at different concentrations against virus when co-incubated with H3N2 virus for 1 minute (third experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | - | + | - | + | + | - | - | + | - |
| | 10⁶ | + | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | - | - | + | - | - | - | + | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | - | + | - | - | + | - | - | + | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| Negative control group (1mM EGCG+culture medium) | 10¹ | * | * | * | * | * | * | * | * | * | * |
| | 10² | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

4) Conclusion on action time of 1 minute: In this experiment, the results of three experiments were statistically analyzed and it was found that when 5mM EGCG was incubated with H3N2 virus for 1 minute, the average inactivation efficiency against virus was 98.11%; when 1mM EGCG was incubated with H3N2 virus for 1 minute, the average inactivation efficiency against virus was 61.78%; and when 0.2mM EGCG was incubated with H3N2 virus for 1 minute, the average inactivation efficiency against virus was 28.93%. In summary, the EGCG treatment could effectively inactivate influenza A H3N2 virus.

### 2. Inactivation efficiency of EGCG against influenza A virus H3N2 within action time of 5 minutes

### 1) Results of the first independent repetition (Table 17):

For the negative control group, cytotoxicity was observed in the 5mM group and 1mM group; the cells in the 0.2mM groups were in good growth status, and there was no virus replication.

For the positive control group, the TCID₅₀/50µL of the dd H₂O+virus group was calculated by the Karber method and was 10^{5.4}.

For the experimental group, virus replication was observed in each experimental group, the TCID₅₀/50µL, of the 5 mM EGCG+virus group was calculated by the Karber method and was 10^{3.8}, the TCID₅₀/50µL of the 1 mM EGCG+virus group was 10^{3.1}, and the TCID₅₀/50µL of the 0.2 mM EGCG+virus group was 10^{4.6}. According to the calculation by the formula, the inactivation efficiency of 5 mM EGCG against the virus was 97.49%, the inactivation efficiency of 1mM EGCG against the virus was 99.50%, and the inactivation efficiency of 0.2 mM EGCG against the virus was 84.15%.

**Table 17 Inactivation effect of EGCG at different concentrations against virus when co-incubated with H3N2 virus for 5 minute (first experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | - | - | - | - | + | - | - | - | - | - |
| | 10⁵ | - | - | - | - | + | - | - | + | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | * | * | * | * | * | * | * | * | * | * |
| | 10³ | + | + | - | - | + | - | - | - | - | - |
| | 10⁴ | + | - | - | + | - | - | - | - | + | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | - | - | - | - | - | - | - | + | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 2) Results of the second independent repetition (Table 18):

For the negative control group, cytotoxicity was observed in 5mM group and 1mM group, the cells in the 0.2mM groups were in good growth status, and there was no virus replication.

For the positive control group, the TCID₅₀/50µL of the dd H₂O+virus group was calculated by the Karber method and was 10^{5.4}.

For the experimental group, virus replication was observed in each experimental group, the TCID₅₀/50µL of the 5 mM EGCG+virus group was calculated by the Karber method and was 10^{1.5}, the TCID₅₀/50µL of the 1 mM EGCG+virus group was 10^{2.5}, and the TCID₅₀/50µL of the 0.2 mM EGCG+virus group was 10^{4.9}. According to the calculation by the formula, the inactivation efficiency of 5 mM EGCG against the virus was 98.74%, the inactivation efficiency of 1mM EGCG against the virus was 99.87%, and the inactivation efficiency of 0.2 mM EGCG against the virus was 68.38%.

**Table 18 Inactivation effect of EGCG at different concentrations against virus when co-incubated with H3N2 virus for 5 minute (second experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | + | * | * | * | * |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | * | * | * | * | * | * | * | * | * | * |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | + | + | - | - | - | - | + | - | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

3) Conclusion on action time of 5 minutes: In this experiment, the results of the two experiments were statistically analyzed and it was found that when 5mM EGCG was incubated with H3N2 virus for 5 minutes, the average inactivation efficiency against virus was 98.32%; when 1mM EGCG was incubated with H3N2 virus for 5 minutes, the average inactivation efficiency against virus was 99.69%; and when 0.2mM EGCG was incubated with H3N2 virus for 5 minutes, the average inactivation efficiency against virus was 76.27%. In summary, the EGCG treatment could effectively inactivate influenza A H3N2 virus.

### Experimental Example 11: Determination of inactivation efficiency of EGCG against influenza B virus B/Lee strain

### (1) Experimental materials

Same as experimental example 9.

### (2) Experimental scheme:

### 1. Treatment of method

According to the molecular weight and mass of EGCG, DEPC water was used to dissolve the compound into a solution with a concentration of 100mM. After the sample was completely dissolved, it was filtered and sterilized with a 0.22µm syringe filter for later use.

### 2. Evaluation of antiviral activity

1) MDCK cells (1.5×10⁴ cells/well) were inoculated into a 96-well plate and cultured overnight.
2) Dilution of compound and action on virus
   ① The final concentration of EGCG was 5 mM: 5 µL of the compound stock solution was added to 95 µL of virus solution, mixed and allowed to stand at room temperature for 1 minute or 5 minutes;
   ② The final concentration of EGCG was 1 mM: 10 µL of the compound stock solution was added to 40 µL of virus culture medium, mixed well, then 5 µL of the mixed solution was taken and added to 95 µL of the virus solution, mixed and allowed to stand at room temperature for 1 minute or 5 minutes;
   ③ The final concentration of EGCG was 0.2 mM: 2 µL of the compound stock solution was added to 48 µL of virus culture medium, mixed well, then 5 µL of the mixed solution was taken and added to 95 µL of the virus solution, mixed and allowed to stand at room temperature for 1 minute or 5 minutes;
   ④ The negative control group only contained the compound at corresponding concentrations and the virus culture medium, the positive control group only contained the corresponding solvent and virus, and other treatment conditions were the same as those of the experimental group.
3) The cell culture medium was discarded, washing was performed three times with 150 µL DPBS, and then 150 µL of the virus culture medium was added to each well.
4) The mixed solution in 2) was diluted ten times in gradient, 50 µL of the dilution solution was add to each well to infect cells, 10 duplicate wells were set up for each dilution solution, and the residual virus titer (TCID₅₀) was determined.
5) After 72 hours of infection, the virus titer was calculated by Karber method.
6) The inactivation efficiency of the sample was calculated by the results of three repetition experiments.

### (3) Experimental results

### 1. Inactivation efficiency of EGCG against influenza B virus B/Lee strain within action time of 1 minute

### 1) Results of the first independent repetition (Table 19):

For the negative control group, cytotoxicity was observed in 5mM group and 1mM group; the cells in the 0.2mM groups were in good growth status, and there was no virus replication.

For the positive control group, the TCID₅₀/50µL of the dd H₂O+virus group was calculated by the Karber method and was 10^{5.7}.

For the experimental group, virus replication was observed in each experimental group, the TCID₅₀/50µL of the 5 mM EGCG+virus group was calculated by the Karber method and was 10^{5.5}, the TCID₅₀/50µL of the 1 mM EGCG+virus group was 10^{5.5}, and the TCID₅₀/50µL of the 0.2 mM EGCG+virus group was 10^{5.5}. According to the calculation by the formula, the inactivation efficiency of 5 mM EGCG against the virus was 36.90%, the inactivation efficiency of 1mM EGCG against the virus was 36.90%, and the inactivation efficiency of 0.2 mM EGCG against the virus was 36.90%.

**Table 19 Inactivation effect of EGCG at different concentrations against virus when co-incubated with B/Lee virus for 1 minute (first experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | - | + | + | + |
| | 10⁶ | - | - | - | - | + | - | - | + | + | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | + | + | + | + | + | + | + | + | + | + |
| | 10⁵ | + | + | + | + | + | + | + | + | + | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | * | * | * | * | * | * | * | * | * | * |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | - | + | + | + | + | + | + | - | + |
| | 10⁶ | - | - | - | - | - | + | + | + | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 2) Results of the second independent repetition (Table 20):

For the negative control group, cytotoxicity was observed in 5mM group and 1mM group; the cells in the 0.2mM groups were in good growth status, and there was no virus replication.

For the positive control group, the TCID₅₀/50µL of the dd H₂O+virus group was calculated by the Karber method and was 10^{5.7}.

For the experimental group, virus replication was observed in each experimental group, the TCID₅₀/50µL of the 5 mM EGCG+virus group was calculated by the Karber method and was 10^{5.3}, the TCID₅₀/50µL of the 1 mM EGCG+virus group was 10^{5.6}, and the TCID₅₀/50µL of the 0.2 mM EGCG+virus group was 10^{5.7}. According to the calculation by the formula, the inactivation efficiency of 5 mM EGCG against the virus was 60.19%, the inactivation efficiency of 1mM EGCG against the virus was 20.57%, and the inactivation efficiency of 0.2 mM EGCG against the virus was 0%.

**Table 20 Inactivation effect of EGCG at different concentrations against virus when co-incubated with B/Lee virus for 1 minute (second experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | - | + | + | + |
| | 10⁶ | - | - | - | - | + | - | - | + | + | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | + | + | + | + | + | + | + | + | + | + |
| | 10⁵ | + | + | + | - | + | + | + | + | - | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| Experimental group (1mM EGCG+virus) | 10¹ | * | * | * | * | * | * | * | * | * | * |
| | 10² | * | * | * | * | * | * | * | * | * | * |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | - | - | - | + | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | + | - | - | - | - | + | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

4) Conclusion on action time of 1 minute: In this experiment, the results of the two experiments were statistically analyzed and it was found that when 5mM EGCG was incubated with B/Lee virus for 1 minute, the average inactivation efficiency against virus was 52.43%; when 1mM EGCG was incubated with B/Lee for 1 minute, the average inactivation efficiency against virus was 28.74%; and when 0.2mM EGCG was incubated with B/Lee virus for 1 minute, the average inactivation efficiency against virus was 18.45%. In summary, the EGCG treatment could effectively inactivate influenza B virus B/Lee strain.

### 2. Inactivation efficiency of EGCG against influenza B virus B/Lee strain within action time of 5 minutes

### 1) Results of the first independent repetition (Table 21):

For the negative control group, cytotoxicity was observed in 5mM group and 1mM group.

For the positive control group, the TCID₅₀/50µL of the dd H₂O+virus group was calculated by the Karber method and was 10^{5.5}.

For the experimental group, virus replication was observed in each experimental group, the TCID₅₀/50µL of the 5 mM EGCG+virus group was calculated by the Karber method and was 10^{5.3}, and the TCID₅₀/50µL of the 1 mM EGCG+virus group was 10^{5.5}. According to the calculation by the formula, the inactivation efficiency of 5 mM EGCG against the virus was 36.90%, and the inactivation efficiency of 1mM EGCG against the virus was 0%.

**Table 21 Inactivation effect of EGCG at different concentrations against virus when co-incubated with B/Lee virus for 5 minute (first experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | - | - | + |
| | 10⁶ | - | - | - | - | + | + | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | + | + | + | - | + | + | + | + | + | + |
| | 10⁵ | - | - | + | + | + | + | + | + | + | - |
| | 10⁶ | - | - | - | - | + | - | - | - | - | - |
| | 10⁷ | - | - | - | - | - | - | + | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| | 10¹ | * | * | * | * | * | * | * | * | * | * |
| Negative control group (5mM EGCG+culture medium) | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | * | * | * | * | * | * | * | * | * | * |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 2) Results of the second independent repetition (Table 22):

For the negative control group, cytotoxicity was observed in 5mM group and 1mM group.

For the positive control group, the TCID₅₀/50µL of the dd H₂O+virus group was calculated by the Karber method and was 10^{5.5}.

For the experimental group, virus replication was observed in each experimental group, the TCID₅₀/50µL of the 5 mM EGCG+virus group was calculated by the Karber method and was 10^{5.3}, and the TCID₅₀/50µL of the 1 mM EGCG+virus group was 10^{5.3}. According to the calculation by the formula, the inactivation efficiency of 5 mM EGCG against the virus was 36.90%, and the inactivation efficiency of 1mM EGCG against the virus was 36.90%.

**Table 22 Inactivation effect of EGCG at different concentrations on virus when co-incubated with B/Lee virus for 5 minute (second experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | - | - | + |
| | 10⁶ | - | - | - | - | + | + | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | * | * |
| | 10⁴ | + | + | + | + | + | + | + | + | + | + |
| | 10⁵ | + | + | + | + | + | + | + | + | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | - | + | - | + | + | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | * | * | * | * | * | * | * | * | * | * |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

3) Conclusion on action time of 5 minutes: In this experiment, the results of the two experiments were statistically analyzed and it was found that when 5mM EGCG was incubated with B/Lee virus for 5 minutes, the average inactivation efficiency against virus was 36.90%; and when 1mM EGCG was incubated with B/Lee virus for 5 minutes, the average inactivation efficiency against virus was 18.45%. In summary, the EGCG treatment could effectively inactivate influenza B virus B/Lee strain.

### Experimental Example 12: Determination of the inactivation efficiency of EGCG against influenza A virus neuraminidase inhibitor-resistant strain ZX-1109

### (1) Experimental materials

Same as experimental example 9.

### (2) Experimental method:

### 1. Treatment of compound

According to the molecular weight and mass of EGCG, DEPC water was used to dissolve the compound into a solution with a concentration of 100mM. After the sample was completely dissolved, it was filtered and sterilized with a 0.22µm syringe filter for later use.

### 2. Evaluation of antiviral activity

1) MDCK cells (1.5 × 10⁴ cells/well) were inoculated into a 96-well plate and cultured overnight.
2) Dilution of compound and action on virus
   ① The final concentration of EGCG was 5 mM: 5 µL of the compound stock solution was added to 95 µL of the virus solution, mixed and allowed to stand at room temperature for 1 minute or 5 minutes;
   ② The final concentration of EGCG was 1 mM: 10 µL of the compound stock solution was added to 40 µL of virus culture medium, mixed well, and then 5 µL of the mixed solution was taken and added to 95 µL of the virus solution, mixed and allowed to stand at room temperature for 1 minute or 5 minutes;
   ③ The final concentration of EGCG was 0.2 mM: 2 µL of the compound stock solution was added to 48 µL of virus culture medium, mixed well, and then 5 µL of the mixed solution was taken and added to 95 µL of the virus solution, mixed and allowed to stand at room temperature for 1 minute or 5 minutes;
   ④ The negative control group only contained the compound at corresponding concentrations and virus culture medium, and the positive control group only contained the corresponding solvent and virus, and other treatment conditions were the same as those of the experimental group.
3) The cell culture medium was discarded, washing was performed three times with 150 µL of DPBS, and then 150 µL of the virus culture medium was added to each well.
4) The mixed solution in 2) was diluted ten times in gradient, 50 µL of the dilution solution was add to each well to infect cells, 10 duplicate wells were set up for each dilution solution, and the residual virus titer (TCID₅₀) was determined.
5) After 72 hours of infection, the virus titer was calculated by Karber method.
6) The inactivation efficiency of the sample was calculated by the results of three repetition experiments.

### (3) Experimental results

### 1. Inactivation efficiency of EGCG against influenza A virus ZX-1109 strain within action time of 1 minute

### 1) Results of the first independent repetition (Table 23):

For the negative control group, cytotoxicity was observed in the 5mM group and in the sample of 10 times dilution in the 1mM group. The cells in other samples in the 1 mM group and the cells in the 0.2 mM group were in good growth status, and there was no virus replication.

For the positive control group, the TCID₅₀/50µL of the dd H₂O+virus group was calculated by the Karber method and was 10^{5.8}.

For the experimental group, virus replication was observed in each experimental group, the TCID₅₀/50µL of the 5 mM EGCG+virus group was calculated by the Karber method and was 10^{4.8}, the TCID₅₀/50µL of the 1 mM EGCG+virus group was 10^{4.6}, and the TCID₅₀/50µL of the 0.2 mM EGCG+virus group was 10^{5.5}. According to the calculation by the formula, the inactivation efficiency of 5 mM EGCG against the virus was 90.00%, the inactivation efficiency of 1mM EGCG against the virus was 93.69%, and the inactivation efficiency of 0.2 mM EGCG against the virus was 49.88%.

**Table 23 Inactivation effect of EGCG at different concentrations against virus when co-incubated with ZX-1109 virus for 1 minute (first experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | - | + | - | - | - | + | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | + | + | + | + | + | + | + | + | + | + |
| | 10³ | + | + | + | + | + | + | * | + | + | + |
| | 10⁴ | + | - | + | + | + | + | - | + | + | + |
| | 10⁵ | - | + | - | + | - | + | + | - | + | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | - | - | - | - | - | - | - | + | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | - | + | - | + | + | + | + | + |
| | 10⁶ | - | - | - | - | - | - | - | + | + | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group | 10¹ | - | - | - | - | - | - | - | - | - | - |
| | 10² | - | - | - | - | - | - | - | - | - | - |
| (0.2mM EGCG+culture medium) | | | | | | | | | | | |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 2) Results of the second independent repetition (Table 24):

For the negative control group, cytotoxicity was observed in the 5mM group and in the sample of 10 times dilution in the 1mM group. The cells in other samples in the 1 mM group and the cells in the 0.2 mM group were in good growth status, and there was no virus replication.

For the positive control group, the TCID₅₀/50µL of the dd H₂O+virus group was calculated by the Karber method and was 10^{5.8}.

For the experimental group, virus replication was observed in each experimental group, the TCID₅₀/50µL of the 5 mM EGCG+virus group was calculated by the Karber method and was 10^{4.6}, the TCID₅₀/50µL of the 1 mM EGCG+virus group was 10^{4.7}, and the TCID₅₀/50µL of the 0.2 mM EGCG+virus group was 10^{5.3}. According to the calculation by the formula, the inactivation efficiency of 5 mM EGCG against the virus was 93.69%, the inactivation efficiency of 1mM EGCG against the virus was 92.06%, and the inactivation efficiency of 0.2 mM EGCG against the virus was 68.38%.

**Table 24 Inactivation effect of EGCG at different concentrations agaist virus when co-incubated with ZX1109 virus for 1 minute (second experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | - | + | - | - | - | + | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | + | * | * | * | + |
| | 10⁴ | + | + | + | - | + | + | + | + | + | - |
| | 10⁵ | - | - | + | - | + | + | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | - | + | - | + | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | + | + | + | + | + | + | + | + | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 3) Results of the third independent repetition (Table 25):

For the negative control group, cytotoxicity was observed in the 5mM group and in the sample of 10 times dilution in the 1mM group. The cells in other samples in the 1 mM group and the cells in the 0.2 mM group were in good growth status, and there was no virus replication.

For the positive control group, the TCID₅₀/50µL of the dd H₂O+virus group was calculated by the Karber method and was 10^{5.8}.

For the experimental group, virus replication was observed in each experimental group, the TCID₅₀/50µL of the 5 mM EGCG+virus group was calculated by the Karber method and was 10^{4.2}, the TCID₅₀/µL of the 1 mM EGCG+virus group was 10⁵, and the TCID₅₀/50µL of the 0.2 mM EGCG+virus group was 10^{5.3}. According to the calculation by the formula, the inactivation efficiency of 5 mM EGCG against the virus was 97.49%, the inactivation efficiency of 1mM EGCG against the virus was 84.15%, and the inactivation efficiency of 0.2 mM EGCG against the virus was 68.38%.

**Table 25 Inactivation effect of EGCG at different concentrations against virus when co-incubated with ZX-1109 virus for 1 minute (third experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | + | - | - | + | - | - | - | - | - |
| | 10⁷ | - | - | - | - | - | - | - | - | + | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | + | * | + | * | * | + | + |
| | 10³ | * | + | * | * | * | * | * | + | * | + |
| | 10⁴ | - | + | + | - | + | + | + | + | + | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | - | - | + | - | + | - | + | + | - |
| | 10⁶ | - | - | - | - | - | - | + | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | - | - | + | + | - | + | + | - | + |
| | 10⁶ | - | - | - | - | - | - | + | - | + | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

4) Conclusion on action time of 1 minute: In this experiment, the results of three experiments were statistically analyzed and it was found that when 5mM EGCG was incubated with ZX1109 virus for 1 minute, the average inactivation efficiency against virus was 93.73%; when 1mM EGCG was incubated with ZX1109 virus for 1 minute, the average inactivation efficiency against virus was 89.97%; and when 0.2mM EGCG was incubated with ZX1109 virus for 1 minute, the average inactivation efficiency against virus was 62.21%. In summary, the EGCG treatment could effectively inactivate influenza A virus ZX1109 strain.

### 2. Inactivation efficiency of EGCG against influenza A virus ZX-1109 strain within action time of 5 minutes

### 1) Results of the first independent repetition (Table 26):

For the negative control group, cytotoxicity was observed in the 5mM group and in the sample of 10 times dilution in the 1mM group. The cells in other samples in the 1 mM group and the cells in the 0.2 mM group were in good growth status, and there was no virus replication.

For the positive control group, the TCID₅₀/50µL of the dd H₂O+virus group was 10^{8.1}.

For the experimental group, virus replication was observed in each experimental group, the TCID₅₀/50µL of the 5 mM EGCG+virus group was calculated by the Karber method and was 10^{5.7}, the TCID₅₀/µL of the 1 mM EGCG+virus group was 10^{6.7}, and the TCID₅₀/50µL of the 0.2 mM EGCG+virus group was 10^{6.3}. According to the calculation by the formula, the inactivation efficiency of 5 mM EGCG against the virus was 99.60%, the inactivation efficiency of 1mM EGCG against the virus was 96.02%, and the inactivation efficiency of 0.2 mM EGCG against the virus was 98.42%.

**Table 26 Inactivation effect of EGCG at different concentrations against virus when co-incubated with ZX-1109 virus for 5 minute (first experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | + | + |
| | 10⁷ | - | + | + | + | + | + | + | + | + | + |
| | 10⁸ | + | - | + | + | + | + | - | - | + | + |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | + | * | * | * | * | * |
| | 10⁴ | + | + | + | - | + | + | + | + | + | + |
| | 10⁵ | + | - | + | + | + | + | - | - | - | - |
| | 10⁶ | + | + | - | - | + | + | + | + | - | - |
| | 10⁷ | + | + | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | + | + | + | * | + | + | * | + | + | + |
| | 10³ | + | + | + | + | + | + | + | + | + | + |
| | 10⁴ | + | + | + | + | + | + | + | + | + | + |
| | 10⁵ | + | + | + | + | + | + | - | + | + | + |
| | 10⁶ | + | + | + | - | - | - | - | - | - | + |
| | 10⁷ | - | - | + | - | - | - | + | + | - | - |
| | 10⁸ | - | - | + | - | + | - | - | + | + | + |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | + | - | + | - | - | - | - | - | - |
| | 10⁷ | - | - | - | - | - | + | - | - | - | - |
| | 10⁸ | - | + | - | + | - | + | - | + | - | + |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group | 10¹ | - | - | - | - | - | - | - | - | - | - |
| | 10² | - | - | - | - | - | - | - | - | - | - |
| (0.2mM EGCG+culture medium) | | | | | | | | | | | |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 2) Results of the second independent repetition (Table 27):

For the negative control group, cytotoxicity was observed in the 5mM group and in the sample of 10 times dilution in the 1mM group. The cells in other samples in the 1 mM group and the cells in the 0.2 mM group were in good growth status, and there was no virus replication.

For the positive control group, the TCID₅₀/50µL of the dd H₂O+virus group was calculated by the Karber method and was 10^{7.9}.

For the experimental group, virus replication was observed in each experimental group, the TCID₅₀/50µL of the 5 mM EGCG+virus group was calculated by the Karber method and was 10^{3.7}, the TCID₅₀/50µL of the 1 mM EGCG+virus group was 10⁶, and the TCID₅₀/50µL of the 0.2 mM EGCG+virus group was 10^{6.2}. According to the calculation by the formula, the inactivation efficiency of 5 mM EGCG against the virus was 99.99%, the inactivation efficiency of 1mM EGCG against the virus was 98.74%, and the inactivation efficiency of 0.2 mM EGCG against the virus was 98.00%.

**Table 27 Inactivation effect of EGCG at different concentrations against virus when co-incubated with ZX-1109 virus for 5 minute (second experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | + | + |
| | 10⁷ | + | - | + | + | - | + | + | + | + | + |
| | 10⁸ | + | + | + | - | + | + | + | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | + | * | * | * | + |
| | 10⁴ | + | - | - | - | - | - | - | - | - | - |
| | 10⁵ | - | - | - | - | - | + | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | - | - | + | + | + | + | + |
| | 10⁶ | + | - | - | - | + | + | + | - | - | - |
| | 10⁷ | - | - | - | - | + | - | - | - | - | - |
| | 10⁸ | - | - | - | - | - | + | + | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | - | + | + | + |
| | 10⁶ | + | - | + | + | - | - | + | - | - | - |
| | 10⁷ | - | + | + | - | - | - | - | - | - | - |
| | 10⁸ | - | - | - | - | + | - | - | + | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 3) Results of the third independent repetition (Table 28):

For the negative control group, cytotoxicity was observed in the 5mM group and in the sample of 10 times dilution in the 1mM group. The cells in other samples in the 1 mM group and the cells in the 0.2 mM group were in good growth status, and there was no virus replication.

For the positive control group, the TCID₅₀/50µL of the dd H₂O+virus group was calculated by the Karber method and was 10^{7.7}.

For the experimental group, virus replication was observed in each experimental group, the TCID₅₀/50µL of the 5 mM EGCG+virus group was calculated by the Karber method and was 10^{3.7}, the TCID₅₀/µL of the 1 mM EGCG+virus group was 10⁶, and the TCID₅₀/µL of the 0.2 mM EGCG+virus group was 10⁷. According to the calculation by the formula, the inactivation efficiency of 5 mM EGCG against the virus was 99.99%, the inactivation efficiency of 1mM EGCG against the virus was 98.00%, and the inactivation efficiency of 0.2 mM EGCG against the virus was 80.05%.

**Table 28 Inactivation effect of EGCG at different concentrations against virus when co-incubated with ZX-1109 virus for 5 minute (third experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | + | + |
| | 10⁷ | - | + | - | - | - | + | - | + | - | + |
| | 10⁸ | + | + | + | + | + | - | + | + | + | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | * | * | * | * | * | * | * | * | * | * |
| | 10³ | * | * | * | * | * | * | * | * | + | * |
| | 10⁴ | - | - | + | + | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | - | - | + | + | + | - | + | - | + |
| | 10⁶ | - | + | - | + | - | + | + | + | + | - |
| | 10⁷ | - | + | - | + | - | + | - | - | - | - |
| | 10⁸ | - | - | - | - | - | - | - | - | + | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | - | + | + | - | + | + | + | + | + |
| | 10⁷ | + | - | - | - | - | - | + | - | + | + |
| | 10⁸ | - | - | - | - | + | - | - | + | - | + |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (SmM EGCG+culture medium) | 10¹ | * | * | * | * | * | * | * | * | * | * |
| | 10² | * | * | * | * | * | * | * | * | * | * |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | * | * | * | * | * | * | * | * | * | * |
| | 10² | - | - | - | - | - | - | - | - | - | - |

| | | | | | | | | | | | |
|---|---|---|---|---|---|---|---|---|---|---|---|
| Group | Dilution factor | 1 | 2 | 3 | 4 | 5 | 6 | 7 | 8 | 9 | 10 |
| Negative control group (0.2mM EGCG+culture medium) | 10¹ | - | - | - | - | - | - | - | - | - | - |
| | 10² | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

4) Conclusion on action time of 5 minute: In this experiment, the results of three experiments were statistically analyzed and it was found that when 5mM EGCG was incubated with ZX1109 virus for 5 minute, the average inactivation efficiency against virus was 99.86%; when 1mM EGCG was incubated with ZX1109 virus for 5 minute, the average inactivation efficiency against virus was 97.59%; and when 0.2mM EGCG was incubated with ZX1109 virus for 5 minute, the average inactivation efficiency against virus was 92.16%. In summary, the EGCG treatment could effectively inactivate influenza A virus ZX1109 strain.

### Experimental Example 13: Determination of inactivation efficiency of EGCG against novel enterovirus EV-D68

### (1) Experimental materials

Same as experimental example 9.

### (2) Experimental method:

### 1. Treatment of compound

According to the molecular weight and mass of EGCG, DEPC water was used to dissolve the compound into a solution with a concentration of 100mM. After the sample was completely dissolved, it was filtered and sterilized with a 0.22µm syringe filter for later use.

### 2. Evaluation of antiviral activity

1) RD cells (1.0×10⁴ cells/well) were inoculated into a 96-well plate and cultured overnight.
2) Dilution of compound and action on virus
   ① The final concentration of EGCG was 5 mM: 5 µL of the compound stock solution was added to 95 µL of the virus solution, mixed and allowed to stand at room temperature for 1 minute or 5 minutes;
   ② The final concentration of EGCG was 1 mM: 10 µL of the compound stock solution was added to 40 µL of virus culture medium, mixed well, and then 5 µL of the mixed solution was taken and added to 95 µL of the virus solution, mixed and allowed to stand at room temperature for 1 minute or 5 minutes;
   ③ The final concentration of EGCG was 0.2 mM: 2 µL of the compound stock solution was added to 48 µL of virus culture medium, mixed well, and then 5 µL of the mixed solution was taken and added to 95 µL of the virus solution, mixed and allowed to stand at room temperature for 1 minute or 5 minutes;
   ④ The negative control group only contained the compound at corresponding concentrations and virus culture medium, and the positive control group only contained the corresponding solvent and virus, and other treatment conditions were the same as those of the experimental group.
3) The cell culture medium was discarded, and 150 µL of the virus culture medium was added to each well.
4) The mixed solution in 2) was diluted ten times in gradient, 50 µL of the dilution solution was add to each well to infect cells, 10 duplicate wells were set up for each dilution solution, and the residual virus titer (TCID₅₀) was determined.
5) After 72 hours of infection, the virus titer was calculated by Karber method.
6) The inactivation efficiency of the sample was calculated by the results of three repetition experiments.

### (3) Experimental results

### 1. Inactivation efficiency of EGCG against novel enterovirus EV-D68 within action time of 1 minute

### 1) Results of the first independent repetition (Table 29):

For the negative control group, cytotoxicity was observed in the sample of 10 times dilution in the 5 mM group. The cells in other samples in the 5 mM group and the cells in other groups were in good growth status, and there was no virus replication.

For the positive control group, virus replication was observed, the TCID₅₀/µL of the ddH₂O+virus group was calculated by the Karber method and was 10^{6.5}.

For the experimental group, virus replication was observed in each experimental group, the TCID₅₀/50µL of the 5 mM EGCG+virus group was calculated by the Karber method and was 10^{5.8}, the TCID₅₀/µL of the 1 mM EGCG+virus group was 10^{6.2}, and the TCID₅₀/50µL of the 0.2 mM EGCG+virus group was 10^{6.5}. According to the calculation by the formula, the inactivation efficiency of 5 mM EGCG against the virus was 80.05%, the inactivation efficiency of 1mM EGCG against the virus was 49.88%, and the inactivation efficiency of 0.2 mM EGCG against the virus was 0.

**Table 29 Inactivation effect of EGCG at different concentrations against virus when co-incubated with EV-D68 virus for 1 minute (first experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | - | + | + |
| | 10⁷ | - | + | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group | 10¹ | * | * | * | * | * | * | * | * | * | * |
| | 10² | + | + | + | + | + | + | + | + | + | + |
| | 10³ | + | + | + | + | + | + | + | + | + | + |
| (5mM EGCG+virus) | 10⁴ | + | + | + | + | + | + | + | + | + | + |
| | 10⁵ | + | + | + | + | + | + | + | + | + | + |
| | 10⁶ | - | + | - | + | - | - | - | - | - | - |
| | 10⁷ | - | - | - | - | - | - | - | - | - | + |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | - | - | - | + | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | - | + | + | + | + | + | - | + |
| | 10⁷ | - | - | + | - | - | - | - | - | - | + |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | | | | | | | | | | |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| (0.2mM EGCG+culture medium) | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 2) Results of the second independent repetition (Table 30):

For the negative control group, cytotoxicity was observed in the sample of 10 times dilution in the 5 mM group. The cells in other samples in the 5 mM group and the cells in other groups were in good growth status, and there was no virus replication.

For the positive control group, virus replication was observed, the TCID₅₀/50µL of the ddH₂O+virus group was calculated by the Karber method and was 10^{6.5}.

For the experimental group, virus replication was observed in each experimental group, the TCID₅₀/50µL of the 5 mM EGCG+virus group was calculated by the Karber method and was 10^{6.0}, the TCID₅₀/50µL of the 1 mM EGCG+virus group was 10^{6.3}, and the TCID₅₀/50µL, of the 0.2 mM EGCG+virus group was 10^{6.3}. According to the calculation by the formula, the inactivation efficiency of 5 mM EGCG against the virus was 68.38%, the inactivation efficiency of 1mM EGCG against the virus was 36.90%, and the inactivation efficiency of 0.2 mM EGCG against the virus was 36.90%.

**Table 30 Inactivation effect of EGCG at different concentrations against virus when co-incubated with EV-D68 virus for 1 minute (second experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | + | - |
| | 10⁷ | - | - | + | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | - | - | + | + | - | - | + | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | - | - | + | - | + | + | + | + | + |
| | 10⁷ | + | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | - | + | + | + | - | - |
| | 10⁷ | - | - | - | - | + | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | | | | | | | | | | |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 3) Results of the third independent repetition (Table 31):

For the negative control group, cytotoxicity was observed in the sample of 10 times dilution in the 5 mM group. The cells in other samples in the 5 mM group and the cells in other groups were in good growth status, and there was no virus replication.

For the positive control group, virus replication was observed, the TCID₅₀/50µL of the ddH₂O+virus group was calculated by the Karber method and was 10^{6.2}.

For the experimental group, virus replication was observed in each experimental group, the TCID₅₀/50µL of the 5 mM EGCG+virus group was calculated by the Karber method and was 10^{5.9}, the TCID₅₀/50µL of the 1 mM EGCG+virus group was 10^{6.0}, and the TCID₅₀/50µL of the 0.2 mM EGCG+virus group was 10^{6.2}. According to the calculation by the formula, the inactivation efficiency of 5 mM EGCG against the virus was 49.88%, the inactivation efficiency of 1mM EGCG against the virus was 36.90%, and the inactivation efficiency of 0.2 mM EGCG against the virus was 0%.

**Table 31 Inactivation effect of EGCG at different concentrations against virus when co-incubated with EV-D68 virus for 1 minute (third experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | - | + | - | + | + | + | + | - | - |
| | 10⁷ | - | - | - | - | - | - | - | + | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | - | - | - | - | - | + | + | + |
| | 10⁷ | + | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | - | - | + | + | - | - | - | + | - |
| | 10⁷ | - | - | - | - | - | - | + | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group | 10¹ | + | + | + | + | + | + | + | + | + | + |
| | 10² | + | + | + | + | + | + | + | + | + | + |
| | 10³ | + | + | + | + | + | + | + | + | + | + |
| (0.2mM EGCG+virus) | 10⁴ | + | + | + | + | + | + | + | + | + | + |
| | 10⁵ | + | + | + | + | + | + | + | + | + | + |
| | 10⁶ | + | + | + | - | - | - | + | + | - | + |
| | 10⁷ | + | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | | | | | | | | | | |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

4) Conclusion on action time of 1 minute: In this experiment, the results of three experiments were statistically analyzed and it was found that when 5mM EGCG was incubated with EV-D68 virus for 1 minute, the average inactivation efficiency against virus was 66.10%; when 1mM EGCG was incubated with EV-D68 virus for 1 minute, the average inactivation efficiency against virus was 41.23%; and when 0.2mM EGCG was incubated with EV-D68 virus for 1 minute, the average inactivation efficiency against virus was 12.30%. In summary, the EGCG treatment could effectively inactivate novel enterovirus EV-D68.

### 2. Inactivation efficiency of EGCG against novel enterovirus EV-D68 within action time of 5 minutes

### 1) Results of the first independent repetition (Table 32):

For the negative control group, cytotoxicity was observed in the sample of 10 times dilution in the 5 mM group. The cells in other samples in the 5 mM group and the cells in other groups were in good growth status, and there was no virus replication.

For the positive control group, virus replication was observed, the TCID₅₀/50µL of the ddH₂O+virus group was calculated by the Karber method and was 10^{6.4}.

For the experimental group, virus replication was observed in each experimental group, the TCID₅₀/50µL of the 5 mM EGCG+virus group was calculated by the Karber method and was 10^{5.8}, the TCID₅₀/50µL of the 1 mM EGCG+virus group was 10^{6.3}, and the TCID₅₀/50µL of the 0.2 mM EGCG+virus group was 10^{6.3}. According to the calculation by the formula, the inactivation efficiency of 5 mM EGCG against the virus was 74.88%, and the inactivation efficiency of 1mM EGCG and 0.2 mM EGCG against the virus was 20.57%.

**Table 32 Inactivation effect of EGCG at different concentrations against virus when co-incubated with EV-D68 virus for 5 minute (first experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH2O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | - | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (SmM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | + | - | + | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | + | - | + | - | + | + | + | + | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | - | + | + | + | - | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (SmM EGCG+culture medium) | 10¹ | * | * | * | * | * | * | * | * | * | * |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | | | | | | | | | | |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 2) Results of the second independent repetition (Table 33):

For the negative control group, cytotoxicity was observed in the sample of 10 times dilution in the 5 mM group. The cells in other samples in the 5 mM group and the cells in other groups were in good growth status, and there was no virus replication.

For the positive control group, virus replication was observed, the TCID₅₀/50µL of the ddH₂O+virus group was calculated by the Karber method and was 10^{6.6}.

For the experimental group, virus replication was observed in each experimental group, the TCID₅₀/50µL of the 5 mM EGCG+virus group was calculated by the Karber method and was 10^{6.0}, the TCID₅₀/50µL of the 1 mM EGCG+virus group was 10^{6.4}, and the TCID₅₀/50µL of the 0.2 mM EGCG+virus group was 10^{6.3}. According to the calculation by the formula, the inactivation efficiency of 5 mM EGCG against the virus was 74.88%, the inactivation efficiency of 1mM EGCG against the virus was 36.90%, and the inactivation efficiency of 0.2 mM EGCG against the virus was 49.88%.

**Table 33 Inactivation effect of EGCG at different concentrations against virus when co-incubated with EV-D68 virus for 5 minute (second experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | + | + |
| | 10⁷ | - | - | + | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | - | - | + | + | - | - | + | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | - | + | + | + | + | + | + | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | + | + | + | - | + | + | + | + | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | | | | | | | | | | |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 3) Results of the third independent repetition (Table 34):

For the negative control group, cytotoxicity was observed in the sample of 10 times dilution in the 5 mM group. The cells in other samples in the 5 mM group and the cells in other groups were in good growth status, and there was no virus replication. ;

For the positive control group, virus replication was observed, the TCID₅₀/50µL of the ddH₂O+virus group was calculated by the Karber method and was 10^{6.6}.

For the experimental group, virus replication was observed in each experimental group, the TCID₅₀/50µL of the 5 mM EGCG+virus group was calculated by the Karber method and was 10^{6.3}, the TCID₅₀/50µL of the 1 mM EGCG+virus group was 10^{6.3}, and the TCID₅₀/50µL of the 0.2 mM EGCG+virus group was 10^{6.4}. According to the calculation by the formula, the inactivation efficiency of 5 mM EGCG against the virus was 49.88%, the inactivation efficiency of 1mM EGCG against the virus was 49.88%, and the inactivation efficiency of 0.2 mM EGCG against the virus was 36.90%.

**Table 34 Inactivation effect of EGCG at different concentrations against virus when co-incubated with EV-D68 virus for 5 minute (third experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | + | + | + | + | + |
| | 10⁷ | - | - | + | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | + | + | + | - | + | + | - | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | - | - | + | + | + | + | + | + | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | + | + | - | + | + | + | + | + | + | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | | | | | | | | | | |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

4) Conclusion on action time of 5 minute: In this experiment, the results of three experiments were statistically analyzed and it was found that when 5mM EGCG was incubated with EV-D68 virus for 5 minutes, the average inactivation efficiency against virus was 66.55%; when 1mM EGCG was incubated with EV-D68 virus for 5 minutes, the average inactivation efficiency against virus was 35.78%; and when 0.2mM EGCG was incubated with EV-D68 virus for 5 minutes, the average inactivation efficiency against virus was 35.78%. In summary, the EGCG treatment could effectively inactivate novel enterovirus EV-D68 strain.

### Experimental Example 14: Determination of inactivation efficiency of EGCG against coronavirus hCoV-229E strain

### (1) Experimental materials

Same as experimental example 9.

### (2) Experimental scheme:

### 1. Treatment of compound

According to the molecular weight and mass of EGCG, DEPC water was used to dissolve the compound into a solution with a concentration of 100mM. After the sample was completely dissolved, it was filtered and sterilized with a 0.22µm syringe filter for later use.

### 2. Evaluation of antiviral activity

1) Huh-7 cells (5000 cells/well) were inoculated into a 96-well plate and cultured overnight.
2) Dilution of compound and action on virus
   ① The final concentration of EGCG was 5 mM: 5 µL of the compound stock solution was added to 95 µL of the virus solution, mixed and allowed to stand at room temperature for 1 minute or 5 minutes;
   ② The final concentration of EGCG was 1 mM: 10 µL of the compound stock solution was added to 40 µL of virus culture medium, mixed well, and then 5 µL of the mixed solution was taken and added to 95 µL of the virus solution, mixed and allowed to stand at room temperature for 1 minute or 5 minutes;
   ③ The final concentration of EGCG was 0.2 mM: 2 µL of the compound stock solution was added to 48 µL of virus culture medium, mixed well, and then 5 µL of the mixed solution was taken and added to 95 µL of the virus solution, mixed and allowed to stand at room temperature for 1 minute or 5 minutes;
   ④ The negative control group only contained the compound at corresponding concentrations and virus culture medium, and the positive control group only contained the corresponding solvent and virus, and other treatment conditions were the same as those of the experimental group.
3) The cell culture medium was discarded and 150 µL of the virus culture medium was added to each well.
4) The mixed solution in 2) was diluted ten times in gradient, 50 µL of the dilution solution was add to each well to infect cells, 10 duplicate wells were set up for each dilution solution, and the residual virus titer (TCID₅₀) was determined.
5) After 120 hours of infection, the virus titer was calculated by Karber method.
6) The inactivation efficiency of the sample was calculated by the results of three repetition experiments.

### (3) Experimental results

### 1. Inactivation efficiency of EGCG against coronavirus hCoV-229E strain with action time of 1 minute

### 1) Results of the first independent repetition (Table 35):

For the negative control group, cytotoxicity was observed in the sample of 10 times dilution in the 5 mM group. The cells in other samples in the 5 mM group and the cells in other groups were in good growth status, and there was no virus replication.

For the positive control group, virus replication was observed, the TCID₅₀/50µL of the ddH₂O+virus group was calculated by the Karber method and was 10^{5.1}.

For the experimental group, virus replication was observed in each experimental group, the TCID₅₀/50µL of the 5 mM EGCG+virus group was calculated by the Karber method and was 10^{4.6}, the TCID₅₀/50µL of the 1 mM EGCG+virus group was 10^{4.6}, and the TCID₅₀/50µL of the 0.2 mM EGCG+virus group was 10^{4.9}. According to the calculation by the formula, the inactivation efficiency of 5 mM EGCG and 1 mM EGCG against the virus was 68.38%, and the inactivation efficiency of 0.2 mM EGCG against the virus was 36.90%.

**Table 35 Inactivation effect of EGCG at different concentrations against virus when co-incubated with hCoV-229E virus for 1 minute (first experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | + | + | - | + | + | - | + | - | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | - | - | - | - | - | - | - | + | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | - | - | - | - | - | - | - | + | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | + | - | - | - | + | + | - | - | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 2) Results of the second independent repetition (Table 36):

For the negative control group, cytotoxicity was observed in the sample of 10 times dilution in the 5 mM group. The cells in other samples in the 5 mM group and the cells in other groups were in good growth status, and there was no virus replication.

For the positive control group, virus replication was observed, the TCID₅₀/50µL of the ddH₂O+virus group was calculated by the Karber method and was 10^{5.2}.

For the experimental group, virus replication was observed in each experimental group, the TCID₅₀/50µL of the 5 mM EGCG+virus group was calculated by the Karber method and was 10^{4.7}, the TCID₅₀/50µL of the 1 mM EGCG+virus group was 10^{4.8}, and the TCID₅₀/50µL of the 0.2 mM EGCG+virus group was 10^{5.0}. According to the calculation by the formula, the inactivation efficiency of 5 mM EGCG against the virus was 68.38%, the inactivation efficiency of 1mM EGCG against the virus was 60.19%, and the inactivation efficiency of 0.2 mM EGCG against the virus was 36.90%.

**Table 36 Inactivation effect of EGCG at different concentrations against virus when co-incubated with hCoV-229E virus for 1 minute (second experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | - | - | + | + | - | + | - |
| | 10⁶ | - | - | - | - | - | - | + | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | + | + | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | - | - | - | + | - | - | + | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | - | - | - | + | - | - | + | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 3) Results of the third independent repetition (Table 37):

For the negative control group, cytotoxicity was observed in the sample of 10 times dilution in the 5 mM group. The cells in other samples in the 5 mM group and the cells in other groups were in good growth status, and there was no virus replication.

For the positive control group, virus replication was observed, the TCID₅₀/50µL of the ddH₂O+virus group was calculated by the Karber method and was 10^{5.6}.

For the experimental group, virus replication was observed in each experimental group, the TCID₅₀/50µL of the 5 mM EGCG+virus group was calculated by the Karber method and was 10^{5.3}, the TCID₅₀/50µL of the 1 mM EGCG+virus group was 10^{5.5}, and the TCID₅₀/50µL of the 0.2 mM EGCG+virus group was 10^{5.5}. According to the calculation by the formula, the inactivation efficiency of 5 mM EGCG against the virus was 49.88%, the inactivation efficiency of 1mM EGCG against the virus was 20.57%, and the inactivation efficiency of 0.2 mM EGCG against the virus was 20.57%.

**Table 37 Inactivation effect of EGCG at different concentrations against virus when co-incubated with hCoV-229E virus for 1 minute (third experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | + | + | + | + | + | + | + | + | + |
| | 10⁶ | - | - | - | - | - | + | + | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | - | + | + | - | - | + | + | + |
| | 10⁶ | - | - | - | - | - | - | - | + | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | - |
| | 10⁶ | - | + | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | - | + | + | + | + | - | + |
| | 10⁶ | - | - | - | + | - | - | - | - | + | - |
| | 10⁷ | - | - | - | - | - | - | - | - | - | - |
| | 10⁸ | - | - | - | - | - | - | - | - | - | - |
| | | | | | | | | | | | |
| Negative control group (5mM EGCG+culture medium) | 10¹ | * | * | * | * | * | * | * | * | * | * |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

4) Conclusion on action time of 1 minute: In this experiment, the results of three experiments were statistically analyzed and it was found that when 5mM EGCG was incubated with hCoV-229E virus for 1 minute, the average inactivation efficiency against virus was 62.21%; when 1mM EGCG was incubated with hCoV-229E virus for 1 minute, the average inactivation efficiency against virus was 49.71%; and when 0.2mM EGCG was incubated with hCoV-229E virus for 1 minute, the average inactivation efficiency against virus was 31.46%. In summary, the EGCG treatment could effectively inactivate coronavirus hCoV-229E strain.

### 2. Inactivation efficiency of EGCG against coronavirus hCoV-229E strain within action time of 5 minutes

### 1) Results of the first independent repetition (Table 38):

For the negative control group, cytotoxicity was observed in the sample of 10 times dilution in the 5 mM group. The cells in other samples in the 5 mM group and the cells in other groups were in good growth status, and there was no virus replication.

For the positive control group, virus replication was observed, the TCID₅₀/50µL of the ddH₂O+virus group was calculated by the Karber method and was 10^{5.6}.

For the experimental group, virus replication was observed in each experimental group, the TCID₅₀/50µL of the 5 mM EGCG+virus group was calculated by the Karber method and was 10^{5.2}, the TCID₅₀/50µL of the 1 mM EGCG+virus group was 10^{5.3}, and the TCID₅₀/50µL of the 0.2 mM EGCG+virus group was 10^{5.5}. According to the calculation by the formula, the inactivation efficiency of 5 mM EGCG against the virus was 60.19%, the inactivation efficiency of 1 mM EGCG against the virus was 49.88%, and the inactivation efficiency of 0.2 mM EGCG against the virus was 20.57%.

**Table 38 Inactivation effect of EGCG at different concentrations against virus when co-incubated with hCoV-229E virus for 5 minute (first experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | - | - | - | - | + | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | - | + | + | + | + | - | + | - | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | - | + | + | - | + | + | - | + |
| | 10⁶ | + | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | - | + | + | + | + | - | + | + | + |
| | 10⁶ | - | - | + | - | - | - | - | - | + | - |
| | 10⁷ | - | - | - | - | - | - | - | - | - | - |
| | 10⁸ | - | - | - | - | - | - | - | - | - | - |
| Negative control group (5mM EGCG+culture medium) | 10¹ | * | * | * | * | * | * | * | * | * | * |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 2) Results of the second independent repetition (Table 39):

For the negative control group, cytotoxicity was observed in the sample of 10 times dilution in the 5 mM group. The cells in other samples in the 5 mM group and the cells in other groups were in good growth status, and there was no virus replication.

For the positive control group, virus replication was observed, the TCID₅₀/50µL of the ddH₂O+virus group was calculated by the Karber method and was 10^{5.6}.

For the experimental group, virus replication was observed in each experimental group, the TCID₅₀/50µL of the 5 mM EGCG+virus group was calculated by the Karber method and was 10^{5.4}, the TCID₅₀/50µL of the 1 mM EGCG+virus group was 10^{5.3}, and the TCID₅₀/50µL of the 0.2 mM EGCG+virus group was 10^{5.5}. According to the calculation by the formula, the inactivation efficiency of 5 mM EGCG against the virus was 36.90%, the inactivation efficiency of 1mM EGCG against the virus was 49.88%, and the inactivation efficiency of 0.2 mM EGCG against the virus was 20.57%.

**Table 39 Inactivation effect of EGCG at different concentrations against virus when co-incubated with hCoV-229E virus for 5 minute (second experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | - | - | - | - | + | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (5mM EGCG+virus) | 10¹ | * | * | * | * | * | * | * | * | * | * |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | - | - | + | - | + |
| | 10⁶ | - | + | - | - | - | - | - | - | - | + |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | - | - | + | + | + | + | + |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | - | + | - | + | + | + | - | + | + | + |
| | 10⁶ | - | + | - | + | - | + | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| Negative control group (1mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (0.2mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

### 3) Results of the third independent repetition (Table 40):

For the negative control group, cytotoxicity was observed in the sample of 10 times dilution in the 5 mM group. The cells in other samples in the 5 mM group and the cells in other groups were in good growth status, and there was no virus replication.

For the positive control group, virus replication was observed, the TCID₅₀/50µL of the ddH₂O+virus group was calculated by the Karber method and was 10^{5.6}.

For the experimental group, virus replication was observed in each experimental group, the TCID₅₀/50µL of the 5 mM EGCG+virus group was calculated by the Karber method and was 10^{5.3}, the TCID₅₀/50µL of the 1 mM EGCG+virus group was 10^{5.4}, and the TCID₅₀/50µL of the 0.2 mM EGCG+virus group was 10^{5.7}. According to the calculation by the formula, the inactivation efficiency of 5 mM EGCG against the virus was 49.88%, the inactivation efficiency of 1mM EGCG against the virus was 36.90%, and the inactivation efficiency of 0.2 mM EGCG against the virus was 0%.

**Table 40 Inactivation effect of EGCG at different concentrations against virus when co-incubated with hCoV-229E virus for 5 minute (third experiment)**

| Group | Dilution factor | 1 | 2 | 3 | 4 | 5 | 6 | 7 | 8 | 9 | 10 |
|---|---|---|---|---|---|---|---|---|---|---|---|
| Positive control group (ddH₂O+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | - | - | - | - | + | - | - | - |
| | 10⁷ | - | - | - | - | - | - | - | - | - | - |
| | 10⁸ | - | - | - | - | - | - | - | - | - | - |
| | | | | | | | | | | | |
| Experimental group (5mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | - | + | - | + | + | - |
| | 10⁶ | - | - | - | - | + | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (1mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | - | + | + | + | - | + | - | + | + |
| | 10⁶ | + | - | - | - | - | - | - | + | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Experimental group (0.2mM EGCG+virus) | 10¹ | + | + | + | + | + | + | + | + | + | + |
| | 10² | + | + | + | + | + | + | + | + | + | + |
| | 10³ | + | + | + | + | + | + | + | + | + | + |
| | 10⁴ | + | + | + | + | + | + | + | + | + | + |
| | 10⁵ | + | + | + | + | + | + | + | + | + | + |
| | 10⁶ | - | - | + | - | - | - | + | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (5mM EGCG+culture medium) | 10¹ | * | * | * | * | * | * | * | * | * | * |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| | | | | | | | | | | | |

| Group | Dilution factor | 1 | 2 | 3 | 4 | 5 | 6 | 7 | 8 | 9 | 10 |
|---|---|---|---|---|---|---|---|---|---|---|---|
| Negative control group (1mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |
| Negative control group (0.2mM EGCG+culture medium) | 10¹ | - | - | - | - | - | - | - | - | - | - |
| | 10² | - | - | - | - | - | - | - | - | - | - |
| | 10³ | - | - | - | - | - | - | - | - | - | - |
| | 10⁴ | - | - | - | - | - | - | - | - | - | - |
| | 10⁵ | - | - | - | - | - | - | - | - | - | - |
| | 10⁶ | - | - | - | - | - | - | - | - | - | - |
| | 10⁷ | - | - | - | - | - | - | - | - | - | - |
| | 10⁸ | - | - | - | - | - | - | - | - | - | - |

| | | | | | | | | | | | |
|---|---|---|---|---|---|---|---|---|---|---|---|
| Note: "+" indicated that CPE test was positive, and there was virus replication in the cell wells; "*" indicated cytotoxicity; "-" indicated that CPE test was negative, and there was no virus replication in the cell wells. | | | | | | | | | | | |

4) Conclusion on action time of 5 minutes: In this experiment, the results of three experiments were statistically analyzed and it was found that when 5mM EGCG was incubated with hCoV-229E virus for 5 minutes, the average inactivation efficiency against virus was 48.99%; when 1mM EGCG was incubated with hCoV-229E virus for 5 minutes, the average inactivation efficiency against virus was 45.55%; and when 0.2mM EGCG was incubated with hCoV-229E virus for 5 minutes, the average inactivation efficiency against virus was 13.71%. In summary, the EGCG treatment could effectively inactivate coronavirus hCoV-229E strain.

### Experimental Example 15: Determination of in vitro bactericidal concentration of EGCG against Helicobacter pylori

In this experimental example, the minimum bactericidal concentration (MBC) of EGCG against *Helicobacter pylori* (Hp) cultured in vitro was determined, and the minimum bactericidal concentration of the fruit-flavored buccal tablets of EGCG of Prescription 4 in Example 14 against Hp was determined.

### Experimental materials:

### Test sample

| Test substance | Character | Molecular formula | Molecular weight |
|---|---|---|---|
| Epigallocatechin gallate (EGCG) | White powder | C₂₂H₁₈O₁₁ | 458.38 |
| Fruit-flavored buccal tablet of EGCG, 5mg EGCG/tablet | Tablet | C₂₂H₁₈O₁₁ | 458.38 |

| | | | |
|---|---|---|---|
| Hp strain: B5-3 Skirrow medium: peptone 15.0g/L, tryptone 2.5g/L, yeast extract 5.0g/L, sodium chloride 5.0g/L (pH7.4) | | | |

### Experimental equipment: three-gas incubator (Thermo3131), microscope (NikonY-TV55), Benchtop centrifuge (Thermo Lengd micro 17R)

### Experiment content:

### 1. Preparation of EGCG storage solution and application solution

### 1.1 Preparation of compound EGCG storage solution

18.3 mg of EGCG powder was accurately weighed and dissolved in 2 mL of deionized water to obtain 20 mM solution, which was sterilized by ultrafiltration for later use (prepared on 20230317, cryopreserved at - 20°C).

### 1.2 Preparation of fruit-flavored buccal tablet of EGCG storage solution

One piece of fruit-flavored buccal tablet of EGCG (EGCG Smg) was taken and dissolved in 1 mL of sterile deionized water, vortexed and mixed for 10 minutes, and there was still white insoluble material. The mixture was heated in water bathing at 50°C for 10 minutes, the white insoluble material was reduced. When the white insoluble material was dissolved, a 8.4mM EGCG suspension with a total volume of 1.3mL was obtained for later use.

### 1.3 EGCG application solution was prepared as follows (doubling dilution)

| Tube No. | Sample volume (µL) | Deionized water (µL) | Concentration (mM) |
|---|---|---|---|
| 1 | 20mM solution, 600 | 600 | 10 |
| 2 | 1# tube, 600 | 600 | 5 |
| 3 | 2# tube, 600 | 600 | 2.5 |

| 1.4 the fruit-flavored buccal tablet of EGCG application solution was prepared as follows (doubling dilution) | | | |
|---|---|---|---|
| Tube No. | Sample volume (µL) | Deionized water (µL) | Concentration (mM) |
| A | 500 | - | 8.4 |
| B | A# tube, 500 | 500 | 4.2 |
| C | B# tube, 500 | 500 | 2.1 |

| | | | |
|---|---|---|---|
| 2. Preparation of Hp bacteria solution | | | |

The B5-3 strain maintained at -80°C was inoculated into Hp liquid medium (8.5mL of Skirrow medium, 0.5mL of sterile 10% glucose, 1mL of calf serum) under microaerobic conditions (10% CO₂, 5% O₂, 85% N₂), incubated at 37°C under shaking for 24 hours, and the OD₆₀₀ value was measured for later use.

### 3. Determination of MBC by broth dilution method

(1) The Hp stock solution in the section 2 above was taken, and inoculated into 10mL of Hp liquid medium at an inoculation volume of 5%, in which EGCG in the following volume was added to the medium, and then incubated at 220rpm under 37°C microaerobic conditions for 24 hours.

| No. | Sampling volume (µL) | Hp culture medium (mL) | Final EGCG concentration (µM) |
|---|---|---|---|
| ① | 1# tube, 400 | 9.6 | 400 |
| ② | 2# tube, 400 | 9.6 | 200 |
| ③ | 3# tube, 400 | 9.6 | 100 |
| P1 | A# tube, 476 | 9.524 | 400 |
| P2 | B# tube, 476 | 9.524 | 200 |
| P3 | C# tube, 476 | 9.524 | 100 |
| Control | - | 10 | 0 |

(2) On the basic of the normal growth of the control bacteria, the turbidity was measured by OD₆₀₀ for comparison to determine whether EGCG had effect on the growth of Hp. Then, the bacterial solution was diluted and spread on a plate for culture and counting, and the bactericidal rate was calculated to determine the minimum bactericidal concentration.

### Experimental results and analysis

### Shake flask culture and plate counting results

7 Bottles of Hp culture medium were cultured in in shake flasks for about 24 hours, then were diluted in gradient to different concentrations and spread in a plate for culture and counting. The results of the experiment showed that EGCG and the fruit-flavored buccal tablet of EGCG showed inactivation effect on Hp in liquid culture medium, and the higher the concentration, the stronger the inactivation effect. EGCG and the fruit-flavored buccal tablet of EGCG cultured in the shake flask at a concentration of 200 µM could inactivate more than 99.9% of Hp, and that at the lowest concentration of 100 µM could inactivate about 98.9%(Table 41).

**Table 41 Count and statistics results of plate spreading**

| No. | Dilution factor | Average number of colonies | Concentration CFU/mL | Bactericidal rate % | EGCG (µM) |
|---|---|---|---|---|---|
| ① | 10⁻⁴ | 22 | 2.2×10⁶ | >99.9 | 400 |
| ② | 10⁻⁴ | 35 | 3.5×10⁶ | >99.9 | 200 |
| ③ | 10⁻⁴ | 142 | 1.4×10⁷ | 98.8 | 100 |
| P1 | 10⁻⁴ | 65 | 6.5×10⁶ | >99.9 | 400 |
| P2 | 10⁻⁴ | 75 | 7.5×10⁶ | >99.9 | 200 |
| P3 | 10⁻⁴ | 130 | 1.3×10⁷ | 98.9 | 100 |
| Control | 10⁻⁶ | 125 | 1.25×10⁹ | - | 0 |

Experimental conclusion: The MBC value of EGCG and fruit-flavored buccal tablets of EGCG against Hp was 200 µM.

## Claims

1. EGCG for use in preventing and/or treating a viral infection or a bacterial infection, wherein EGCG is administered buccally, and the residence time of EGCG in the buccal cavity is greater than or equal to 1 minute.

2. The EGCG for use according to claim 1, wherein the residence time of EGCG in the buccal cavity is 1 to 20 minutes, preferably 1 to 5 minutes.

3. The EGCG for use according to claim 1 or 2, wherein the average concentration of EGCG in the buccal cavity is 0.2 to 16mM, preferably 0.2 to 15.5mM, such as 0.2 to 15.28mM, 0.2 to 15mM, 0.2 to 12mM, 0.2 to 10mM, 0.2 to 8mM, 0.2 to 6mM, 0.2 to 5mM, 0.2 to 4mM, 0.2 to 2mM.

4. The EGCG for use according to any one of claims 1-3, wherein the EGCG exist in the form of a buccal tablet.

5. The EGCG for use according to any one of claims 1-4, wherein the virus infection is an infection caused by a respiratory virus or other virus that is transmitted through respiratory tract.

6. The EGCG for use according to any one of claims 1-5, wherein the virus infection is an infection caused by a virus selected from the group consisting of influenza A virus, influenza B virus, rhinovirus (HRV), adenovirus (AdV), coronavirus (including but not limited to SARS virus, SARS-CoV-2), vesicular stomatitis virus (VSV), human immunodeficiency virus (HIV), enterovirus 71 (EV71), hepatitis B virus (HBV), herpes simplex virus type 1 and 2 (HSV-1 and HSV-2), hepatitis C virus (HCV), coxsackievirus, and novel enterovirus D68 (EV-D68) ; preferably, the virus infection is an infection caused by a SARS-CoV-2.

7. The EGCG for use according to any one of claims 1-3, wherein the bacterium infection is an infection caused by a *Helicobacter pylori.*

8. A buccal tablet comprising EGCG, a filler, a lubricant, and a flavoring agent, wherein the EGCG content in each buccal tablet is 0.3 mg to 100 mg, preferably, the buccal tablet further compriseing a coloring agent, wherein, the coloring agent is preferably a food coloring and preferably, the buccal tablet further comprising an adhesive.

9. The buccal tablet according to claim 8, **characterized by** one or more of the following items:
1) the filler is one or more selected from the group consisting of cyclodextrin, lactose, mannitol, sorbitol, glycine, microcrystalline cellulose, starch, skimmed milk powder, collagen and dextrin,
2) the cyclodextrin is one or more selected from the group consisting of α-cyclodextrin, β-cyclodextrin, γ-cyclodextrin and pharmaceutically acceptable cyclodextrin derivative; preferably, the pharmaceutically acceptable cyclodextrin derivative is one or more selected from the group consisting of dimethyl-β-cyclodextrin, 2-hydroxyethyl-β-cyclodextrin, 2-hydroxypropyl-β-cyclodextrin, 3-hydroxypropyl-β-cyclodextrin, sulfobutyl ether-[3-cyclodextrin and trimethyl-β-cyclodextrin; preferably, the cyclodextrin is one or more selected from the group consisting of β-cyclodextrin, dimethyl-β-cyclodextrin, 2-hydroxyethyl-β-cyclodextrin, 2-hydroxypropyl-β-cyclodextrin, 3-hydroxypropyl-β- cyclodextrin, sulfobutyl ether-[3-cyclodextrin and trimethyl-β-cyclodextrin,
3) the lubricant is one or more selected from the group consisting of magnesium stearate, talc, and silicon dioxide,
4) the flavoring agent is one or more selected from the group consisting of sweetening agent, menthol, fruity flavoring agent, and souring agent; preferably, the flavoring agent comprises sweetening agent, menthol; preferably, the flavoring agent comprises sweetening agent, menthol, and fruity flavoring agent; preferably, the flavoring agent comprises sweetening agent, menthol, and souring agent; preferably, the flavoring agent comprises sweetening agent, menthol, fruity flavoring agent, and souring agent; preferably, the sweetening agent is one or more selected from the group consisting of sucrose, sucralose, glucose, aspartame, xylitol, sorbose, fructose, fructo-oligosaccharides and stevioside; preferably, the sweetening agent is fructo-oligosaccharides or a combination of fructo-oligosaccharides and other sweetening agent, and the other sweetening agent is one or more selected from the group consisting of sucrose, sucralose, glucose, aspartame, xylitol, fructose and stevioside; preferably, the souring agent is one ore more selected from the group consisting of L-malic acid, D-malic acid, and DL-malic acid,
5) the adhesive is one or more selected from the group consisting of sodium carboxymethylcellulose, methylcellulose, povidone, tragacanth and gum arabic.

10. The buccal tablet according to claim 8 or 9, wherein the EGCG content in each buccal tablet is 0.5 mg to 50 mg, such as 0.625 mg, 1.25 mg, 2.5 mg, 5 mg, 7.5 mg, 10 mg, 25 mg, or 50 mg, preferably, the EGCG content in each buccal tablet is 2.5 mg to 7.5 mg or 5 mg to 10 mg.

11. The buccal tablet according to any one of claims 8-10, wherein the residence time of the buccal tablet in the buccal cavity is greater than or equal to 1 minute, preferably, the residence time of the buccal tablet in the buccal cavity is 1-20 minutes, 1 to 5 minutes, 5 to 15 minutes or 15 to 20 minutes.

12. The buccal tablet according to any one of claims 8-11, wherein the dissolution of the buccal tablet is greater than 70% in 15-20 minutes when measured by the paddle method at 37°C and 100 rpm in an aqueous medium.

13. The buccal tablet according to any one of claims 8-12, **characterized by** one or more of the following items:
1) the filler content in each buccal tablet is 20 to 200 times, such as30 to 150 times, 40 to 100 times, 40 to 160 times, 60 to 120 times, 80 to 140 times, the EGCG content,
2) the lubricant content in each buccal tablet is 0.1 to 5.0% (preferably 0.5 to 3.5%, further preferably 1 to 3%, more preferably 1.5 to 2.5%) by weight of the buccal tablet;
3) the flavoring agent content in each buccal tablet is 0.5 to 100 times, preferably 1 to 100 times, more preferably 1 to 50 times, even more preferably 1 to 15 times, 5 to 25 times, 10~35 times or 5~20 times, the EGCG content;
preferably, the flavoring agent comprises sweetening agent and menthol, wherein the menthol content in each buccal tablet is 0.01 to 5 times,preferably 0.1 to 4 times, 0.2 to 3 times, 0.01 to 1.5 times, 0.05 to 1 times, 0.1 to 0.75 times, the EGCG content;
preferably, the flavoring agent further comprises a fruity flavoring agent, wherein the fruity flavoring agent content in each buccal tablet is no more than 30 times the EGCG content, preferably no more than 25 times the EGCG content, preferably no more than 20 times the EGCG content; for example, 15 times or 10 times the EGCG content,
preferably, the flavoring agent further comprises a souring agent, wherein the souring agent content in each buccal tablet is 0.01 to 7 times, preferably 0.01 to 1.5 times, 0.05 to 1 times, 0.1 to 1 times, 0.05 to 4 times, 0.1 to 2 times, the EGCG content,
preferably, the sweetening agent comprises fructo-oligosaccharides, wherein the fructo-oligosaccharides content in each buccal tablet is 2 to 25 times, preferably 3 to 20 times, more preferably 4 to 15 times, the EGCG content,
4) the adhesive content in each buccal tablet is 0.1 to 5.0%, preferably 0.5 to 3.5%, and more preferably 0.5 to 2.5%, by weight of the buccal tablet.

14. The buccal tablet according to claim 8, comprising:
| | |
|---|---|
| EGCG | 2.0% by weight |
| Filler | 91.0% by weight |
| Lubricant | 2.0% by weight |
| Flavoring agent | 3.0% by weight |
| Adhesive comprising: | 2.0% by weight, or |
| EGCG | 2.0% by weight |
| Filler | 89.5% by weight |
| Lubricant | 2.0% by weight |
| Flavoring agent | 4.5% by weight |
| Adhesive comprising: | 2.0% by weight, or |
| EGCG | 1.0% by weight |
| Filler | 50.4% by weight |
| Lubricant | 2.0% by weight |
| Flavoring agent | 45.6% by weight |
| Adhesive comprising: | 1.0% by weight, or |
| EGCG | 1.0% by weight |
| Filler | 88.4% by weight |
| Lubricant | 2.0% by weight |
| Flavoring agent | 7.6% by weight |
| Adhesive comprising: | 1.0% by weight, or |
| EGCG | 1.0% by weight |
| Filler | 87.9% by weight |
| Lubricant | 2.0% by weight |
| Flavoring agent | 7.6% by weight |
| Adhesive | 1.0% by weight |
| Food coloring comprising: | 0.5% by weight, or |
| EGCG | 1.0% by weight |
| Filler | 87.0% by weight |
| Lubricant | 2.0% by weight |
| Flavoring agent | 9.5% by weight |
| Food coloring comprising: | 0.5% by weight, or |
| EGCG | 1.0% by weight |
| Filler | 84.2% by weight |
| Lubricant | 2.0% by weight |
| Flavoring agent | 12.3% by weight |
| Food coloring comprising: | 0.5% by weight, or |
| Sorbitol | 60.48% by weight |
| Fruity flavoring agent | 15.00% by weight |
| Skimmed milk powder | 15.00% by weight |
| Fructo-oligosaccharides | 5.00% by weight |
| EGCG | 0.83% by weight |
| Magnesium stearate | 2.00% by weight |
| Aspartame | 0.20% by weight |
| Sucralose | 0.50% by weight |
| DL-malic acid | 0.80% by weight |
| Menthol | 0.10% by weight |
| Food coloring or comprising: | 0.09% by weight |
| Sorbitol | 49.38% by weight |
| Fruity flavoring agent | 20.00% by weight |
| Skimmed milk powder | 20.00% by weight |
| Fructo-oligosaccharides | 5.00% by weight |
| EGCG | 0.83% by weight |
| Magnesium stearate | 3.00% by weight |
| Aspartame | 0.20% by weight |
| Sucralose | 0.60% by weight |
| DL-malic acid | 0.80% by weight |
| Menthol | 0.10% by weight |
| Food coloring or comprising: | 0.09% by weight |
| Sorbitol | 74.30% by weight |
| Fruity flavoring agent | 9.00% by weight |
| Skimmed milk powder | 8.00% by weight |
| Fructo-oligosaccharides | 5.00% by weight |
| Magnesium stearate | 2.00% by weight |
| EGCG | 0.71% by weight |
| Sucralose | 0.25% by weight |
| DL-malic acid | 0.50% by weight |
| Aspartame | 0.10% by weight |
| Menthol | 0.05% by weight |
| Food coloring or comprising: | 0.09% by weight |
| EGCG | 0.7% to 1.5% by weight |
| Sorbitol | 77% to 82% by weight |
| Fructo-oligosaccharides | 3% to 7% by weight |
| Sucralose | 0.3% to 1.5% by weight |
| Menthol | 0.2% to 0.4% by weight |
| DL-malic acid | 0.3% to 1% by weight |
| Aspartame | 1% to 2% by weight |
| Magnesium stearate | 1% to 3% by weight |
| Fruity flavoring agent | 8% to 10% by weight |
| Food coloring | 0.02% to 0.2% by weight. |

15. Use of the buccal tablet according to any one of claims 8 to 14 in the manufacture of a product for preventing and/or treating a viral infection or a bacterial infection,
preferably, the virus infection is an infection caused by a respiratory virus or other virus that is transmitted through respiratory tract,
preferably, the virus infection is an infection caused by a virus selected from the group consisting of influenza A virus, influenza B virus, rhinovirus (HRV), adenovirus (AdV), coronavirus (including but not limited to SARS virus, SARS-CoV-2), vesicular stomatitis virus (VSV), human immunodeficiency virus (HIV), enterovirus 71 (EV71), hepatitis B virus (HBV), herpes simplex virus type 1 and 2 (HSV-1 and HSV-2), hepatitis C virus (HCV), coxsackievirus, novel enterovirus D68 (EV-D68);
preferably, the virus infection is an infection caused by a SARS-CoV-2;
preferably, the bacterium infection is an infection caused by a *Helicobacter pylori.*
